# EUROPEAN PATENT SPECIFICATION

(11) **EP 2 170 360 B1**
(45) Date of publication and mention of the grant of the patent: **28.08.2013**
(21) Application number: 08763636.1
(22) Date of filing: 26.06.2008
(51) Int. Cl.: A01N 65/00, A61K 36/605

(54) **HERBAL COMPOSITIONS FOR THE TREATMENT OF DIABETES AND/OR CONDITIONS ASSOCIATED THEREWITH**
PFLANZLICHE ZUSAMMENSETZUNGEN ZUR BEHANDLUNG VON DIABETES UND/ODER ERKRANKUNGEN IM ZUSAMMENHANG DAMIT
COMPOSITIONS DE PLANTES POUR LE TRAITEMENT DU DIABÈTE ET/OU DE MALADIES ASSOCIÉES À CELUI-CI

(30) Priority: 28.06.2007 IL 18431207
(43) Date of publication of application: 07.04.2010
(73) Proprietor: Ascarit Ltd, 54017 Givat Shmuel (IL)
(72) Inventor: FOGEL, Dov, 56510 Savyon (IL)
(74) Representative: Turner, Craig Robert
(86) International application number: PCT/IL2008/000880
(87) International publication number: WO 2009/001362

(56) References cited:
- WO-A1-99/20289
- US-A1- 2003 206 976
- US-A1- 2004 161 524
- US-A1- 2007 122 492
- GOLALIPOUR MOHAMMAD JAFAR ET AL: "The protective activity of Urtica dioica leaves on blood glucose concentration and beta-cells in streptozotocin-diabetic rats", PAKISTAN JOURNAL OF BIOLOGICAL SCIENCES, ASIAN NETWORK FOR SCIENTIFIC INFORMATION (A NSINE T), PK, vol. 10, no. 8, 15 April 2007 (2007-04-15) , pages 1200-1204, XP009143116, ISSN: 1028-8880
- KAVALALI G ET AL: "Hypoglycemic activity of Urtica pilulifera in streptozotocin-diabetic rats.", February 2003 (2003-02), JOURNAL OF ETHNOPHARMACOLOGY FEB 2003 LNKD- PUBMED:12648821, VOL. 84, NR. 2-3, PAGE(S) 241 - 245, XP002616912, ISSN: 0378-8741 * abstract *
- FARZAMI BIJAN ET AL: "Induction of insulin secretion by a component of Urtica dioica leave extract in perifused Islets of Langerhans and its in vivo effects in normal and streptozotocin diabetic rats.", November 2003 (2003-11), JOURNAL OF ETHNOPHARMACOLOGY NOV 2003 LNKD- PUBMED:14522431, VOL. 89, NR. 1, PAGE(S) 47 - 53, XP002616913, ISSN: 0378-8741 * abstract *
- BNOUHAM MOHAMED ET AL: "Antihyperglycemic activity of the aqueous extract of Urtica dioica.", December 2003 (2003-12), FITOTERAPIA DEC 2003 LNKD- PUBMED:14630172, VOL. 74, NR. 7-8, PAGE(S) 677 - 681, XP002616914, ISSN: 0367-326X * abstract *
- ONAL SEÇIL ET AL: "Inhibition of alpha-glucosidase by aqueous extracts of some potent antidiabetic medicinal herbs", PREPARATIVE BIOCHEMISTRY AND BIOTECHNOLOGY, DEKKER, NEW YORK, NY, US, vol. 35, no. 1, 1 January 2005 (2005-01-01), pages 29-36, XP009143245, ISSN: 1082-6068
- ZHANG JI ET AL: "Hypoglycaemic effect of Artemisia sphaerocephala Krasch seed polysaccharide in alloxan-induced diabetic rats", SWISS MEDICAL WEEKLY, EMH SWISS MEDICAL PUBLISHERS, BASEL, CH, vol. 136, no. 33-34, 19 August 2006 (2006-08-19), pages 529-532, XP009143246, ISSN: 1424-7860
- KORKMAZ H ET AL: "Effect of Artemisia santonicum L. on blood glucose in normal and alloxan-induced diabetic rabbits.", November 2002 (2002-11), PHYTOTHERAPY RESEARCH : PTR NOV 2002 LNKD- PUBMED:12410552, VOL. 16, NR. 7, PAGE(S) 675 - 676, XP002616915, ISSN: 0951-418X * abstract *
- RIBNICKY ET AL: "Antihyperglycemic activity of Tarralin(TM), an ethanolic extract of Artemisia dracunculus L", PHYTOMEDICINE, GUSTAV FISCHER VERLAG, STUTTGART, DE, vol. 13, no. 8, 11 September 2006 (2006-09-11), pages 550-557, XP005607004, ISSN: 0944-7113, DOI: DOI:10.1016/J.PHYMED.2005.09.007
- JUNG ET AL: "The anti-diabetic effects of ethanol extract from two variants of Artemisia princeps Pampanini in C57BL/KsJ-db/db mice", FOOD AND CHEMICAL TOXICOLOGY, PERGAMON, GB, vol. 45, no. 10, 22 May 2007 (2007-05-22), pages 2022-2029, XP022196764, ISSN: 0278-6915, DOI: DOI:10.1016/J.FCT.2007.04.021
- ANDALLU B ET AL: "EFFECT OF MULBERRY (MORUS INDICA L.) THERAPY ON PLASMA AND ERYTHROCYTE MEMBRANE LIPIDS IN PATIENTS WITH TYPE 2 DIABETES", CLINICA CHIMICA ACTA, ELSEVIER BV, AMSTERDAM, NL, vol. 314, no. 1/02, 1 December 2001 (2001-12-01), pages 47-53, XP001104055, ISSN: 0009-8981, DOI: DOI:10.1016/S0009-8981(01)00632-5
- MUDRA MITCHELL ET AL: "Influence of mulberry leaf extract on the blood glucose and breath hydrogen response to ingestion of 75 g sucrose by type 2 diabetic and control subjects.", May 2007 (2007-05), DIABETES CARE MAY 2007 LNKD- PUBMED:17303787, VOL. 30, NR. 5, PAGE(S) 1272 - 1274, XP002616916, ISSN: 1935-5548 * the whole document *
- VERSPOHL EUGEN J ET AL: "Antidiabetic effect of Cinnamomum cassia and Cinnamomum zeylanicum in vivo and in vitro.", March 2005 (2005-03), PHYTOTHERAPY RESEARCH : PTR MAR 2005 LNKD- PUBMED:15934022, VOL. 19, NR. 3, PAGE(S) 203 - 206, XP002616917, ISSN: 0951-418X * abstract *
- PETLEVSKI R ET AL: "Effect of 'antidiabetis' herbal preparation on serum glucose and fructosamine in NOD mice", JOURNAL OF ETHNOPHARMACOLOGY, ELSEVIER SCIENTIFIC PUBLISHERS LTD, IE, vol. 75, no. 2-3, 1 May 2001 (2001-05-01), pages 181-184, XP027380323, ISSN: 0378-8741 [retrieved on 2001-05-01]
- KULTUR ET AL: "Medicinal plants used in Kirklareli Province (Turkey)", JOURNAL OF ETHNOPHARMACOLOGY, ELSEVIER SCIENTIFIC PUBLISHERS LTD, IE, vol. 111, no. 2, 5 April 2007 (2007-04-05) , pages 341-364, XP022021930, ISSN: 0378-8741, DOI: DOI:10.1016/J.JEP.2006.11.035
- KONNO ET AL.: 'Mulberry latex rich in antidiabetic sugar-mimic alkaloids forces dieting on caterpillars' PROC. NATL. ACAD. SCI. (US) vol. 103, no. 5, 31 January 2006, pages 1337 - 1341, XP008131952
- RASTOGI ET AL.: 'Antimycobacterial Activity of Chemically Defined Natural Substances from the Caribbean Flora in Guadeloupe' FEMS IMMUNOL. & MED. MICROBIOL. vol. 20, no. 4, 17 January 2006, pages 267 - 273, XP008131953
- KONG ET AL.: 'Parental Use of the Term "Hot Qi" to Describe Symptoms in Their Children in Hong Kong: a Cross Sectional Survey "Hot Qi" in Children' J. ETHNOBIOL. ETHNOMED. vol. 2, no. 2, 05 January 2006, pages 1 - 7, XP008148166

## Description

### FIELD AND BACKGROUND OF THE INVENTION

The present invention relates to the field of therapeutic natural products, particularly to plant extracts effective in treating and preventing diabetes and/or conditions associated therewith.

Diabetes mellitus is a common, serious disease characterized by hyperglycemia. The World Health Organization (WHO) estimates that more than 190 million people worldwide have diabetes and this number is constantly on the rise. The disease can be divided into two major subclasses: insulin-dependent diabetes mellitus (IDDM), also known as type I diabetes, and non-insulin-dependent diabetes mellitus (NIDDM), also known as type II diabetes or adult diabetes.

In adult (type II) diabetes, high levels of sugar in the blood cause a recognizable increase in the osmolarity of the blood, causing confusion and difficult conditions such as unconsciousness. Long-term complications usually appear in both small and large blood vessels, affecting the heart, kidneys and eyes, as well as damaging the sensory nerves (especially in the legs). Damage also occurs in the autonomic nervous system, and is expressed by impotence, infertility and disturbances in the digestive system, heart and blood vessels.

A significant proportion of diabetic adults also suffer from high blood pressure. Both of these diseases must be attended to because both accelerate degeneration with complications, especially in the blood vessels and the heart. 41 million Americans are suffering from pre-diabetes, a precursor to diabetes type II. Another condition associated with diabetes as a secondary cause, is hypertriglyceridemia, a commonly encountered lipid abnormality, which is frequently associated with additional lipid and metabolic derangements.

The regulation of diet and exercise and/or treatment with insulin or hypoglycemia drugs have been used for control of both diabetes and triglycerides. Treatment with these agents is successful in some cases, but the mortality index continues to rise. Insulin treatment and Hypoglycemic agents (such as Sulfonylureas, Biguanides and alpha glucosidase inhibitors) provide symptomatic relief rather than a cure for diabetes and are associated with side effects. The side effects of Sulfonylureas include hypoglycemia, renal and heptic disease, gastrointestinal disturbance, increase cardiovascular mortality, dizziness, drowsiness headache and others. The major side effects of Biguanides are lactic acidosis and increased cardiocvascular mortality. The side effects of alpha glucosidase inhibitors include gastrointestinal side effects and hypoglycemia.

In addition to conventional treatments relying on insulin injections or oral medications, natural products, including plant materials, have been reportedly tried in alternative treatments of conditions such as diabetes.

Various Mulberry species (Moraceae family) are known for therapeutical effects. For example, the branches and bark of *M*. *alba* (white Mulberry) are used for lowering blood pressure (Enkhmaa et al., J. nutr. 2005, vol. 135, no. 4, pp. 729-734). *M. indica L.* leaves have been shown to possess hypoglycemic, hypotensive, and diuretic properties, although there was no apparent effect on the concentrations of the glycosylated hemoglobin (Hb A(1)c) in diabetic patients (Andallu et al., Clin Chim Acta. 2001 Dec; 314 (1-2):47-53). It is important to note that presently known Mulberry-derived compounds have been derived from fruits, bark, and in the case of leaves, have avoided extracting the leaf in a way that would include the latex covering the leaves.

*Canella winteriana* (known by the common names: Canella, Cinnamon Bark, Cinnamonbark, Pepper Cinnamon, Wild Cinnamon) and *Cinnamomum cassia* (Cinnamon) have been suggested to be useful in maintaining healthy blood sugar levels, and cholesterol levels as well (Aaron W. Jensen, "Cinnamon Reduces Blood Sugar and Cholesterol Levels", InsuLife™. But, while daily doses of cinnamon did produce some significant reductions in blood sugar levels, total cholesterol levels, triglyceride levels and even lower levels of LDL lipoproteins, the higher dose of cinnamon did not seem to improve the actual reduction in the various serum levels mentioned above when compared to the lower doses of cinnamon (Alam et al., Diabetes Care 26:3215-3218, 2003).

*Artemisia* is a large, diverse genus of plants with between 200-400 species belonging to the daisy family Asteraceae, and grow in temperate climates, usually in dry or semi-dry habitats. US Patent No. 6,350,478 discloses extracts of *Artemisia judaica* and fractions thereof, and has found that some fractions of are insulinomemetic and others have glucagon antagonistic properties.

The stinging nettle (*Urtica dioica*) is the best known member of the *Urtica* genus, which has been widely used by herbalists around the world for centuries. In Brazil and Peru herbal medicine the entire plant is used for various disorders, including diabetes and inflammatory conditions. Farzami et al. (Journal of Ethnopharmacology 2003; 89:47-53) disclosed the induction of insulin secretion by a component of *Urtica* dioica leaf extract and its in vivo effects in diabetic rats.

A systematic review of herbs and dietary supplements for glycemic control in diabetes has been conducted by Yeh et al. (Diabetes Care. 2003 Apr, 26(4):1277-94). This review has concluded that the heterogeneity and small number of studies per supplement precluded formal meta-analyses and that thus there is still insufficient evidence to draw definitive conclusions about the efficacy of individual herbs and supplements for diabetes although the researchers have indicated that they appear to be generally safe. The best evidence for efficacy from adequately designed randomized controlled trials (RCTs) is available for Coccinia indica and American ginseng.

In another study on the efficacy of dietary supplementation with botanicals on carbohydrate metabolism in humans (Cefalu et al., Endocr. Metab Immune Disord Drug Targets, 2008, 8(2):78-81), the efficacy of Bitter Melon (*Momordica charantia*)*,* Fenugreek (*trigonella foenum graecum*)*, Gymnema Sylvestre,* Ivy Gourd (*Coccinia indica*)*,* Nopal or Prickly Pear Cactus (*Opuntia streptacantha*)*,* Ginseng, Aloe Vera, Russian Tarragon (*Artemisia dracunculus*)*,* and Garlic (*Allium sativum)* was evaluated and the researchers have concluded that there is insufficient evidence from clinical studies for any of the botanicals reviewed, and thus that it is premature to actively recommend use of any particular herb to treat either glucose or other risk factors.

Thus, there is an ongoing need to find natural remedies for diabetes and/or for hypertriglyceridemia, which exhibit high efficiency in lowering the glucose and/or triglyceride levels in the blood, even in clinically tested experiments.

### SUMMARY OF THE INVENTION

According to one aspect of the present invention there is provided a herbal composition comprising:
at least one *Urtica* species or an extract thereof,
at least one *Artemisia* species or an extract thereof, and
an extract of at least one *Morus* species,
wherein the extract of a *Morus* species is prepared of *Morus* leaves and comprises *Morus* latex.

According to further features in preferred embodiments of the invention described below, the *Morus* species is selected from the group consisting of *Morus alba, Morus bombycis, Morus indica, Morus insignis, Morus nigra* and *Morus Australis,* and any combinations thereof.

According to still further features in the described preferred embodiments, the *Urtica* species is selected from the group consisting of *Urtica dioica, Urtica urens* and *Urtica pilulifera,* and any combinations thereof.

According to still further features in the described preferred embodiments, the *Artemisia* species is selected from the group consisting of *Artemisia dracunculus, Artemisia herba alba, Artemisia pallens Wall, Artemisia roxburghiana* and *Artemisia judaica,* and any combinations thereof.

According to still further features in the described preferred embodiments, the amount of the *Morus* extract ranges from about 10 weight percentage to about 90 weight percentages of the total weight of the composition, an amount of the *Artemisia* species ranges from about 1 weight percentage to about 50 weight percentages of the total weight of the composition, and an amount of the *Urtica* species ranges from about 2 weight percentage to about 50 weight percentages of the total weight of the composition.

Preferably, the amount of the *Morus* extract ranges from about 50 weight percentage to about 90 weight percentages of the total weight of the composition, an amount of the *Artemisia* species ranges from about 1 weight percentage to about 20 weight percentages of the total weight of the composition, and an amount of the *Urtica* species ranges from about 2 weight percentage to about 20 weight percentages of the total weight of the composition.

According to still further features in the described preferred embodiments, the composition described herein further comprises at least one species selected from a *Cinnamomum* species, a *Canella* species, a *Taraxacum* species and/or a *Rosa* species.

According to still further features in the described preferred embodiments, the *Cinnamomum* species is selected from the group consisting of *Cinnamomum cassia, Cinnamomum zeylanicum; Cinnamomum saigonicum, Cinnamomum aromaticum* and *Cinnamomum laurus.*

According to still further features in the described preferred embodiments, the *Canella* species is *Canella winterana.*

According to still further features in the described preferred embodiments, the *Taraxacum* species is *Taraxacum Officinale.*

According to still further features in the described preferred embodiments; the *Rosa* species is *Rosa canina.*

According to still further features in the described preferred embodiments, the amount of the *Morus* extract ranges from about 50 weight percentage to about 90 weight percentages of the total weight of the composition, an amount of the *Artemisia* species ranges from about 1 weight percentage to about 20 weight percentages of the total weight of the composition, an amount of the *Urtica* species ranges from about 2 weight percentage to about 20 weight percentages of the total weight of the composition, and an amount of any species selected from a *Cinnamomum* species, a *Canella* species, a *Taraxacum* species and/or a *Rosa* species ranges from about 5 weight percentages to about 30 weight percentages of the total weight of the composition.

According to still further features in the described preferred embodiment, the composition described herein further comprises one or more species selected from a *Humulus* species, a *Gymnema* species, a *Trigonella* species, a *Punica* species, a *Salix* species, and/or an *Olea* species.

According to a preferred embodiment of the present invention, the composition described herein further comprises at least one carrier.

According to still further features in the described preferred embodiments, the composition described herein is in the form of a tea, a tincture, a concoction, an infusion a tablet, a capsule, a pill, a bar, a chewable gum, a lotion, a powder or granules.

According to still further features in the described preferred embodiments, there is provided a dietary composition comprising a herbal composition described herein and a dietetically acceptable excipient.

Preferably, this composition is a dietary supplement.

According to still further features in the described preferred embodiments, there is provided a pharmaceutical composition comprising a herbal composition described herein and a pharmaceutically acceptable excipient.

According to still further features in the described preferred embodiments, there is provided a food product which includes a composition as described herein.

According to still further features in the described preferred embodiments, the compositions described herein are packaged in a packaging material and identified in print, in or on the packaging material, for use in the treatment and/or prevention of diabetes and/or conditions associated therewith.

According to still further features in the described preferred embodiments, the compositions described herein are packaged in a packaging material and identified in print, in or on the packaging material, for use in the treatment and/or prevention of dyslipidemia.

According to another aspect of the present invention there is provided a use of the compositions described herein, in the preparation of a medicament for treating and/or preventing diabetes and/or conditions associated therewith.

According to another aspect of the present invention there is provided a use of the compositions described herein, in the preparation of a medicament for treating and/or preventing dyslipidemia.

According to further features in preferred embodiments of the invention described below, there is provided the use of a composition described herein in the preparation of an orally-administrable composition.

According to still further features in the described preferred embodiments, the composition is a food supplement.

According to still further features in the described preferred embodiments, the composition is a pharmaceutical composition.

Also disclosed herein is a method of treating and/or preventing diabetes and/or conditions associated therewith, the method comprising administering to a subject in need thereof a therapeutically effective amount of any of the compositions described herein.

Preferably, diabetes is type II diabetes.

According to further features in preferred embodiments of the invention described below, the type II diabetes associated conditions are selected from atherosclerosis, hypertension, diabetic retinopathy, diabetic nephropathy, diabetic polyneuropathies, thyroid disorders, leg ulcers, diabetic foot, liver diseases, kidney function, sight, impotence and constipation.

According to still further features in the described preferred embodiments, the method comprises administering to a subject in need thereof a therapeutically effective amount of any of the compositions described herein.

According to still further features in the described preferred embodiments, the therapeutically effective amount ranges from about 1000 mg per adult per day to about 6 grams per adult per day. Preferably, the therapeutically effective amount ranges from about 1500 mg per adult per day to about 3 grams per adult per day.

According to still another aspect of the present invention there is provided a process of preparing a herbal composition comprising:
at least one *Urtica* species or an extract thereof,
at least one *Artemisia* species or an extract thereof, and
an extract of at least one *Morus* species,
wherein the extract of a *Morus* species is prepared of *Morus* leaves and comprises *Morus* latex;
wherein said extract of at least one *Morus* species is prepared by:
   a) cutting or grinding fresh washed leaves of at least one *Morus* species to obtain cut or ground fresh *Morus* leaves;
   b) letting the cut or ground fresh *Morus* leaves stand until latex is exuded therefrom;
   c) pressing the leaves to obtain a fresh juice and squeezed leaves;
   d) collecting the juice;
   e) brewing the juice to obtain a brewed juice;
   f) chilling and filtering the brewed juice to obtain a liquid extract of at least one *Morus* species.

According to further features in preferred embodiments of the invention described below, the process described herein further comprises, subsequent to pressing the leaves, collecting the squeezed leaves into sacks, and adding the sacks containing squeezed leaves to the juice before brewing
According to still further features in the described preferred embodiments, the process described herein further comprises concentrating the juice before brewing it.

According to still further features in the described preferred embodiments, the process described herein further comprises drying the liquid extract to obtain a solid extract of at least one *Morus* species.

According to still further features in the described preferred embodiments, the process described herein further comprises sieving the solid extract, to obtain a powder extract of at least one *Morus* species.

According to still another aspect of the present invention there is provided a process for preparing a herbal composition as described herein comprising: preparing an extract of at least one *Morus* species as described herein, and mixing thereto at least one *Urtica* species or an extract thereof, and at least one *Artemisia* species or an extract thereof.

According to still another aspect of the present invention there is provided a process for preparing a herbal composition as described herein comprising: preparing an extract of at least one *Morus* species as described herein, and mixing thereto at least one *Urtica* species or an extract thereof, at least one *Artemisia* species or an extract thereof, and at least one species selected from a *Cinnamomum* species, a *Canella* species, a *Taraxacum* species and/or a *Rosa* species or extracts thereof.

According to still another aspect of the present invention there is provided a process for preparing a herbal composition as described herein comprising: preparing an extract of at least one *Morus* species as described herein, and mixing thereto af least one *Urtica* species or an extract thereof, at least one *Artemisia* species or an extract thereof, at least one species selected from a *Cinnamomum* species, a *Canella* species, a *Taraxacum* species and/or a *Rosa* species or extracts thereof, and at least one species selected from a *Humulus* species, a *Gymnema* species, a *Trigonella* species, a *Punica* species, a *Salix* species, and/or an *Olea* species or extracts thereof.

### BRIEF DESCRIPTION OF THE DRAWINGS

In the drawings:
FIG. 1 presents Average Fasting Blood glucose levels (mg/dL) on 26 diabetic patients after 3 months treatment with liquid Sugar Red formulation, according to a preferred embodiment of the present invention; and
FIG. 2 presents HbA1C levels in 22 diabetic patients after 3 months treatment with liquid Sugar Red formulation, according to a preferred embodiment of the present invention.

### DESCRIPTION OF THE PREFERRED EMBODIMENTS

The present invention relates to herbal compositions and their use in treating and/or preventing diabetes and related conditions, and promoting normal blood sugar levels. Further, the anti-diabetic herbal compositions of this invention are also effective in the treatments of insulin dependent (type I) diabetes and non insulin dependent (type II) diabetes.

The present invention also relates to herbal compositions and their use in treating and/or preventing dyslipidemia, and promoting normal lipids levels.

As disclosed in the Background section hereinabove, there is an ongoing attempt to find natural solutions to treating diabetes and conditions associated thereto. Although many folk medicines are based on some herb or combination of herbs, there is no clear clinical proof of their effectiveness and safety.

It has now been found by the present inventors that a combination of specially-extracted *Morus* leaves with an *Urtica* herb or extract and an *Artemisia* herb or extract, results in obtaining an effective and safe anti-diabetic composition.

As shown in the Examples section which follow, this combination acts synergistically and provides an improved method to treat and/or prevent diabetes, as compared to presently known drugs and as compared to the commonly known effect of these herbs.

This effect has been surprisingly provided by extracting fresh *Morus* leaves in such a way that the *Morus* latex is contained in the *Morus* extract which forms part of the composition.

Thus, according to one aspect of the invention there is provided a herbal composition comprising:
at least one *Urtica* species or an extract thereof,
at least one *Artemisia* species or an extract thereof, and
an extract of at least one *Morus* species, wherein the extract of the *Morus* species is prepared of fresh *Morus leaves.*

The term "herbal composition" is used interchangeably with the term "herb composition" and refers to any composition derived from a plant source. As used herein, the term "herb" or "medicinal herb" will be utilized to denote medicinal plants, or selected portions of such plants.

The *Morus* species can be selected from the group consisting of *Morus alba, Morus bombycis, Morus indica, Morus insignis, Morus nigra* and *Morus Australis,* and any combinations thereof. For example, successful results were obtained from a combination of *Morus alba* and *Morus nigra.*

The *Urtica* species can be selected from the group consisting of *Urtica dioica, Urtica urens* and *Urtica pilulifera,* and any combinations thereof.

The *Artemisia* species can be selected from the group consisting of *Artemisia dracunculus, Artemisia herba alba, Artemisia pallens Wall, Artemisia roxburghiana* and *Artemisia judaica,* and any combinations thereof.

As noted herein, the compositions of the present invention are based on mixtures of the *Morus* special extract with either one or more parts of the plants in a milled form or their extracts.

It is to be noted that the term "extract" is used herein to include all of the many types of preparations containing some or all of the active ingredients found in the relevant plants.

The effective parts of the plant useful for the extraction process preferably include seeds, leaves, stems, flowers, roots, berries and barks.

Thus the extracts may be produced by cold extraction techniques using a variety of different extraction solvents including, but not limited to, water, fatty solvents (such as olive oil), glycols, CO₂, and hydro-alcoholic solvents (e.g. 70 % ethanol). Cold extraction techniques are usefully applied to softer parts of the plant such as leaves and flowers, or in cases wherein the desired active components of the plant are heat labile.

Alternatively, the aforementioned solvents may be used to produce extracts of the desired plants by a hot extraction technique, wherein said solvents are heated to a high temperature, the precise value of said temperature being dependent on the properties of the chosen solvent or the desired plant, and maintained at that temperature throughout the extraction process. Hot extraction techniques are more commonly applied to the harder, tougher parts of the plant, such as bark, woody branches and larger roots. In some cases, sequential extractions need to be performed in more than one solvent, and at different temperatures.

Standard procedures for producing plant extracts (including hot extraction, cold extraction and other techniques) are described in many publications including "Medicinal plants: a field guide to the medicinal plants of the Land of Israel (in Hebrew), author: N. Krispil, Har Gilo, Israel, 1986"; "Making plant medicine, author: R. Cech, pub. by Horizon Herbs, 2000"; "The Healing Powers of Herbs", M. Murry, 2nd edition, Prima Health, 1995; Potter's New cyclopedia of botanical drugs & preparations, R.C. Wren, Potter's Limited 1988, 1994; The Encyclopedia of Medicinal Plants, A. Chevallier, Dorling Kindersley, 1996; The Master Book of Herbalism, P. Beyerl, Phenix Publishing Inc., 1984.

As is well known in the field of botany, when cutting *Morus* leaves, a latex is exuded by the plant, as a deterrent to most insects. Commonly known methods of preparing extracts of *Morus* leaves have circumvented the inclusion of this latex in the extract. For example:
■ Boiling or drying the leaves before cutting them, which a common technique used to better preserve the leaves during processing, transportation and storage, prevents latex formation. Therefore, dried or boiled leaves will not exude any latex into a later formed extract.
■ Thoroughly rinsing cut leaves (even fresh leaves) before further processing thereof washes away any latex that has been exuded, and again the formed extract will not contain any latex.

In contrast, the present inventors have used freshly cut leaves, and have deliberately let the leaves stand after cutting or grinding them, to allow complete secretion of this latex into the *Morus* extract.

It was found that this specially-formed *Morus* extract, in combination with herbs or extracts of *Urtica* or *Artemisia* species, showed synergistic activity in lowering blood glucose levels and in lowering blood lipid concentrations, while forming a safe and stable herbal composition, which was successfully tested on a variety of diabetic patients.

Thus, according to another aspect of the invention, there is provided a process of preparing an extract of at least one *Morus* species, the process comprising:
a) cutting or grinding fresh leaves of at least one *Morus* species to obtain cut or ground fresh *Morus* leaves;
b) letting the cut or ground fresh *Morus* leaves stand until latex is exuded therefrom;
c) pressing the leaves to obtain a fresh juice and squeezed leaves;
d) collecting the juice;
e) brewing the juice to obtain a brewed juice;
f) chilling and filtering the brewed juice to obtain a liquid extract of at least one *Morus* species.

Fresh leaves are used for the process of the present invention. The term "fresh" as used herein refers to leaves that have maintained their turgor, as can be easily assessed visually before processing: a fresh leaf is a leaf that is still green and appears to have a flat, non-crumbled or wrinkled texture, which is similar to the appearance of the leaf on the plant before collection of the leaf. This is achieved either by processing the leaves as soon as they are collected, or alternatively by collecting the leaves and keeping them under optimal cooling and humidity conditions, as determined by persons skilled in the art, and depending on the specific plant. In most cases, a herb can maintain its turgor under optimal preserving conditions for about a month.

It has further been found by the inventors that young *Morus* leaves are preferable to older leaves. These young leaves can be found at the higher parts of the tree.

Since it is undesirable to wash the leaves after cutting them, as this may remove any exuded latex, the leaves are preferably washed by tap water prior to cutting them, thereby cleaning the leaves and preparing them for further processing. It is important that hot or boiling water will not be used as washing water in order to avoid damage to the latex exuded by the plant.

The cutting or grinding of the leaves can be done by a number of commonly used techniques, either manually or automatically.

The cut or ground fresh leaves are let to stand to allow complete secretion of this latex into the *Morus* extract. Preferably, standing time is at least 20 minutes, more preferably at least 30 minutes in the open air.

Pressing of the cut or ground leaves is conducted by conventional screw pressing techniques to obtain a fresh juice and a residual dry material of squeezed leaves.

The term "brewing" as used herein refers to boiling or simmering. The present inventors have studied the effects of brewing time on the anti-diabetic activity of the extract and have found considerable differences in lowering blood glucose, with the most effective inhibition observed when 8 to 10 minutes brewing time. This time may vary depending on the environmental conditions but should not exceed 20 minutes without adversely affecting the properties of the extract. These results are further supported by Hansawasdi and Kawabata who studied α-glucosidase inhibitory effect of *Morus* alba leaves (Fitoterapia. 2006 Dec; 77(7-8): 568-73).

Following the brewing of the juice, the brewed juice is quickly chilled to room temperature (15-40 °C more preferably 20-30 °C) in order to avoid degradation and contamination, and is filtered to obtain a clear liquid extract of an at least one *Morus* species.

In order to produce an optimally extracted product, it is possible to collect the squeezed leaves left after the initial pressing stage, put them into sacks or bags, and add these sacks or bags to the juice just before brewing it, thereby extracting any residual constituents still present in the pressed leaves.

Furthermore, it is possible to concentrate the obtained juice prior to brewing it, for easier processing.

Following brewing, the brewed juice is filtered by any number of conventional techniques, to obtain a liquid extract.

A herbal dry extract may be obtained by further drying of the liquid form of the extract. Thus, according to a preferred embodiment of the present invention, this process further comprises drying the liquid *Morus* extract to obtain a solid extract thereof, by means of spray drying, vacuum oven drying, fluid-bed drying or freeze-drying.

It was further possible to sieve this solid extract, thereby obtaining a powder extract of the *Morus* leaves.

The extract may be used in liquid form or in a dry form, and may be mixed with other liquid or solid herbal extracts, as shown in the Examples section.

Thus, according to another aspect of the invention, there is provided a process of preparing the composition of *Morus* extract, *Artemisia* herb or extract and *Urtica* herb or extrcat, by first preparing the *Morus* extract described herein, and then mixing thereto at least one *Urtica* species or an extract thereof, and at least one *Artemisia* species or an extract thereof.

As shown in the examples which follow, one preferable composition includes *Morus* alba, *Morus* nigra, *Urtica* dioica, and *Artemisia arborescens.*

The composition described herein has been shown to have anti-diabetic, further lowering triglyceride and cholesterol levels in the blood.

This was achieved in compositions comprising from about 10 weight percentage to about 90 weight percentages of the *Morus* extract described herein, (preferably from about 50 weight percentage to about 90 weight percentages), from about 1 weight percentage to about 50 weight percentages of the *Artemisia* species (preferably from about 1 weight percentage to about 20 weight percentages) and from about 2 weight percentage to about 50 weight percentages of the *Urtica* species (preferably, from about 2 weight percentage to about 20 weight percentages). All weight percentages refer to the total weight of the composition.

The inventors have also found that compositions formed from adding at least one species selected from a *Cinnamomum* species; a *Canella* species, a *Taraxacum* species and/or a *Rosa* species, to these three herbs produced compositions which successfully, and synergistically, lowered blood glucose levels and blood lipids levels.

These compositions were prepared in a process similar to that described for the preparation of the *Morus, Urtica* and *Artemisia* compositions described hereinabove, by mixing the additional herbs or extracts with the specially prepared *Morus* extract.

Thus, according to a preferred embodiment of the present invention, there is provided the composition described herein, further comprising at least one species selected from a *Cinnamomum* species, a *Canella* species, a *Taraxacum* species and/or a *Rosa* species.

The *Cinnamomum* species can be selected from the group consisting of *Cinnamomum cassia, Cinnamomum zeylanicum, Cinnamomum saigonicum, Cinnamomum aromaticum and Cinnamomum laurus.*

Preferably, the *Canella* species is *Canella winterana.*

Further preferably, the *Taraxacum* species is *Taraxacum Officinale.*

Yet further preferably, the *Rosa* species is *Rosa canina.*

In addition to the amounts of *Morus, Artemisia* and *Urtica* described above, these any of the additional species *(Cinnamomum, Canella, Taraxacum* and/or a *Rosa*) was successfully used in an amount ranging from about 5 weight percentages to about 30 weight percentages of the total weight of the composition.

One especially successful such composition is termed "Sugar Red" and comprises:
The *Morus alba*/*nigra* specially prepared extract, *Taraxacum officinale, Urtica dioica , Artemisia arborescens, Cinnamomum cassia* and *Rosa canina .*

These herbs are present in effective amounts of from about 50 weight percentages to about 75 weight percentages of *Morus alba*/*nigra* specially prepared extract, from about 5 weight percentages to about 15 weight percentages of *Taraxacum officinale,* from about 5 weight percentages to about 15 weight percentages of *Urtica dioica,* from about 5 weight percentages to about 15 weight percentages of *Artemisia arborescens,* from about 5 weight percentages to about 15 weight percentages of *Cinnamomum cassia* and from about 5 weight percentages to about 15 weight percentages of *Rosa canina.*

Tests carried out on NIDDM patients, who have been suffering for many years and were being treated with conventional medicines, have shown a notable improvement rate in symptoms associated with diabetes as well as kidney function, sight, impotence and prevention of constipation.

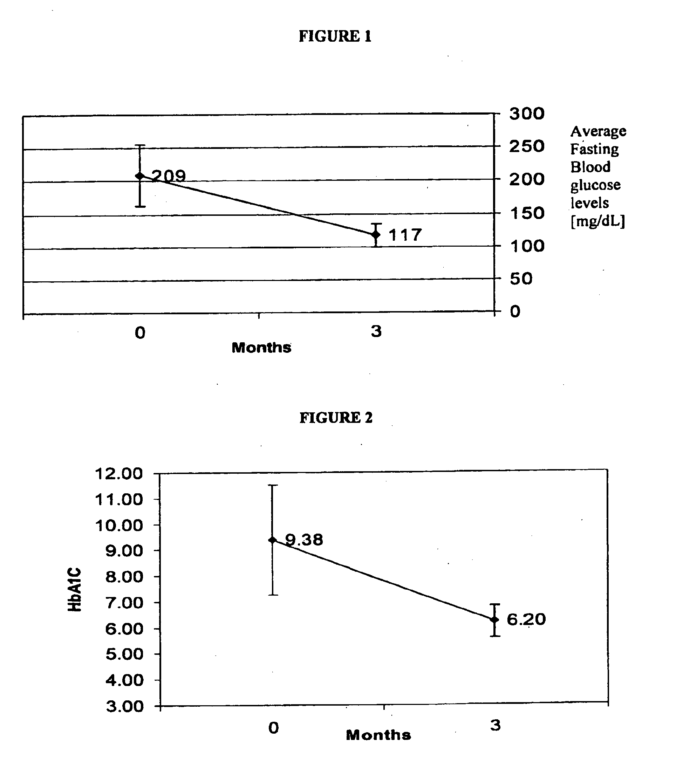

All groups using the compositions of the invention, such as the "Sugar Red" composition, showed significant glycemic control improvement (see Figure 1 and Examples 15-20). The Sugar Red composition also lowered levels of triglycerides and cholesterol in the patients' blood (see Figure 2). The results obtained using Sugar-Red as an infusion or capsule were highly effective in relieving hypoglycemia, showing a descent in glucose levels combined by a major drop in AlC levels (by 33.94% ± 12.66%, see Figure 2). AlC is a test (also know as glycated hemoglobin for HbAlc) that provides an estimate of average blood glucose control for the past 3 months Thus, this result in for itself shows the higher efficacy of the present compositions comparatively to other commercially known drugs.

Optionally, the composition described herein further comprises one or more species selected from a *Humulus* species, a *Gymnema* species, a *Trigonella* species, a *Punica* species, a *Salix* species, and/or an *Olea* species. These additional herbs or extracts are added in amounts which range from about 5 weight percentages to about 30 weight percentages of the total weight of the composition.

These compositions were prepared in a process similar to that described for the preparation of the *Morus, Urtica* and *Artemisia* compositions described hereinabove, by mixing the additional herbs or extracts with the specially prepared *Morus* extract.

The composition of the present invention may further include at least one compound selected from the group consisting of Abelmoschus moschatus, Abies pindrow, Abroma augusta, Acacia arabica , Acanthopanax senticosus , Acer ginnala, Achillea millefolium, Achyranthes aspera, Achyrocline satureioides, Acosmium panamense, Acourtia thurberi , Adhatoda vasica, Aegle marmelose, Agaricus bisporus, Aglaonema treubii, Agrimony eupatoria, Ajuga iva, Alchemilla vulgaris, alfalfa, Allium cepa, Allium-sativum , Aloe barbadensis, aloe vera, Aloe vera, alpha-lipoic acid, alpha-lipoic acid salts, Anacardium occidentale, andrographis paniculata, Andrographis paniculata, Anemarrhena asphodeloides, Angylocalyx pynaertii, Annona squamosa, Apocynum venetum , Aralia cortex, Aralia dasyphylla , Arctium lappa, Arctostaphylos uva-ursi, Areca catechu, Arfazetin, Artocarpus heterophyllus , Asteracanthus longifolia, Astragalus, Averrhoa bilimbi Linn, Azadirachta indica, Azorella compacta, Bacopa monniera , Baja California Norte, banaba leaf, Bauhinia candicans, Bauhinia forficata, Bauhinia variegate, Benincasa hispida Thunb , Berberis vulgaris , Bergenia himalacia, beta glucan, Beta vulgaris , Bidens pilosa, bilberry, Biophytum sensitivum, bitter melon, Bixa orellana, Boerhaavia diffusa, Boswellia carteri, Brassica juncea, Brassica oleracea var. botrytis, Bryonia alba, Buddleja officinalis, Bumelia sartorum, Caesalpinia bonducella, Caesalpinia ferrea , Cajanus cajan , Calamintha officinalis Moench, Camellia sinensis , Capparis decidua, Capparis spinosa, Capsicum frutescens, Caralluma edulis, Carissa edulis , carnosine, Carum carvi , Casearia esculenta, Cassia alata, Cassia auriculata, Cassia fistula, Cassia occidentalis, Cassia tora, Castanospermum australe, Catharanthus roseus, Catharanthus roseus Linn, Cecropia obtusifolia, Centaurea aspera, Centaurea corcubionensis, Centaurea corcubionensis, Centaurea iberica, Centaurium umbellatum Gilib, Cephalandra indica, Chamaemelum nobile, Chelidonium majus, chromium picolinate, Chrysanthemum leucanthemum, Chrysobalanus icaco, Cicer aretinum, Cichorium intybus , Cimicifuga dahurica, Cinnamomum cassia, Cinnamomum tamala, Cinnamomum zeylanicum, Cirsium pascuarense, Cissus sicyoides, Citrullus colocynthis, Citrus limon, Clausena anisata, Cleome-Droserifolia , Cnidium officinale Makino , Cnidoscolus chayamansa, Coccinia indica, Coenzyme Q10, coffee berry, Cogniauxia podoleana Baillon, Colocassia esculenta, Commelina communis, Commiphora myrrha , Convallaria majalis, Convolvulus althaeoides, Coptis chinensis , Coriandrum sativum, Corni fructus, Comus macrophylla, Comus officinalis , Comus stolonifera, corosolic acid, Coscinium fenestratum , Costus speciosus, Cressa cretica, Crotalaria aegyptiaca, Croton cajucara, Cryptolepis sanguinolenta, Cucumis sativus, Cucurbita ficifolia, Cuminum cyminum, Cuminum nigrum, Curcuma longa, Cyamopsis tetragonalobus , Cyclocarya paliurus, Cymbopogon proximus , damiana leaves, Daucus carrota, dehydroepiandrosterone (DHEA), Dendrobium candidum, Dendrocalamus hamiltonii, Dioscorea cayenensis, Dioscorea dumetorum, Dioscoreae rhizoma, D-pinitol, Eclipta alba, Emblica officinalis, Enicostemma littorale , Ephedra alata, Epidendrum monsenii, Equisetum myriochaetum, Erigeron breviscapus, Eriobotrya japonica, Eruca sativa, Erythroxylum coca, Eucalyptus globulus, Eugenia jambolana, Euphorbia helioscopia, Euphorbia prostrata , Euphrasia officinale, fenugreek, Ferula asafoetida, Ferula persica, fiber, Ficus bengalensis , Ficus carica , Ficus glomerata, Ficus racemosa, Ficus relegiosa, Filipendula ulmaria, Fraxinus excelsior, Fumaria parviflora, Galega officinalis, Garcinia kola, garlic, Gentiana olivieri, gingko biloba, Ginkgo biloba, Globularia alypum , Globularia alypum, Glycine max , Glycyrhizza glabra, Glycyrrhiza uralensis , Glycyrrhizae radix, goat's tue, Gongronema latifolium, green tea, Guaiacum coulteri, Guazuma ulmifolia, Gymnema montanum, gymnema sylvestre, Gymnema sylvestre, Gynura procumbens, Gypsum Fibrosum, Haloxylon salicornicum, Hamamelis virginiana , Hamiltonia suaveolens, Harpagophytum procumbens , hawthorn, Helicteres isora, Hericium erinaceus, Hintonia latiflora, Hintonia standleyana, Hordeum vulgare, Hovenia dulcis Thunb , Humulus lupulus, Hydnocarpus wightiana, Hydrastis canadensis, Hygrophila longifolia, Hylocereus undatus, Hypoxis hemerocallidea, Ibervillea sonorae, Illicium religiosum, Indigofera arrecta, Indigofera macrophylla , Inula racemosa, Ipomoea aquatica, Irvingia gabonesis , jambul seeds, Jatropha curcas , Juniperus communis, Kalopanax pictus, Lactuca sativa var. romana, Lagerstroemia speciosa, Larrea tridentata, Laurus nobilis , Lavandula dentata , Lavandula stoechas , Leguminous, Lepechinia caulescens, Lepidium sativum, Leucas lavandulaefolia Rees, lex guayusa, Loranthus begwensis, Luffa aegyptiaca , Lupinus albus, Lupinus termis, Lycium shawii, Lygos raetam Forssk, Lygos raetam var. bovei, Lygos raetam var. sarcocarpa , Magnesium stevia, maitake mushroom, Mangifera indica, Marrubium vulgare, Medicago sativa, Melia azadirachta, Mentha piperitae, momordica charantia, Momordica charantia, Momordica foetida, Monstera deliciosa, Morinda lucida Benth, Moringa oleifera, Moringa stenopetala, Moutan radicis, Mucuna pruriens, Murraya koenigii, Musa sapientum, Myrcia multiflora, Myrcia uniflora , Myristica fragrens , Myrtus communis, Nelumbo nucifera, Nephrolepsis tuberosa, niacin, Nigella sativa, Ocimum canum, Ocimum gratissimum, Ocimum sanctum, Olea europaea, Olea ferruginea, olive leaf, omega 3 fatty acids, Opuntia ficus indica Mill., Opuntia megacantha, Opuntia robusta, Opuntia streptacantha, Origanum syriacum, Origanum vulgare, Otholobium pubescens , Paeonia lactiflora, Panax ginseng, Panax notoginseng, Panax quinquefolium, Panax quinquefolius L, Pandanus odorus , Pantoea agglomerans, Parmentiera edulis, Paronychia argentea Lam , Pedilanthus tithymaloides, Peganum harmala, Pergularia tomentosaPhaseolus mungo, Phaseolus aureus , Phaseolus vulgaris, Phellinus baumii, Phellodendron cortex , phosphatidylserine, Phyllanthus amarus , Phyllanthus sellowianus, Phytolacca americana, Pimenta dioica, Piper sarmentosum, pipsissewa, Pistacia atlantica, Plantago psyllium, Plectranthus rugosus, Polygala senega, Polygonatum officinale, Portulaca oleracae , portulaca oleracea, Poupartia birrea , Premna integrifolia, Prunus davidiana, Psacalium decompositum, Psacalium peltatum , Psidium guajava, Psoralea corylifolia, pterocarpus marsupium, Pterocarpus marsupium, Pueraria lobata, Pueraria thunbergiana, Punica granatum, Pycnanthus angolensis Warb, pycnogenol, Quercus alba, Rehmanniae radix, Retama raetam, Rhazya stricta, Rheedia gardneriana, Rhizoma alismatis, Rhizophora mangle, Rhus hirta, Rubus fructicosis, Rubus imperialis, Rubus ulmifolius, Ruscus aculeatus, Salacia oblonga, salacia reticulata, Salacia reticulata, Salvia aegyptiaca, Salvia avandulifolia, Salvia coccinia, Salix alba, Salvia officinale, Sambucus nigra, Sanguis draxonis, Schisandra chinensis, Sclerocarya birrea, Scoparia dulcis , Scrophularia deserti , Securigera securidaca, Sesamum indicum, Sesbenia aegyptiaca, Silybum marianum, silymarin, Smallanthus sonchifolius, Solanum lycocarpum, Spergularia purpurea, Spinacea oleracea, Stephania hernandifolia, Stevia rebaudiana Bertoni, Suaeda fruticosa, Sutherlandia frutescens, sweet sumach, Swertia chirayita, Syzgium aromaticum , Syzygium alternifolium, Syzygium aromaticum, Syzygium cordatum, Syzygium cumini , Syzygium jambos , Tabanus fulvus, Tamarindus indica , Taraxacum officinale, Telfaria occidentalis, Telfaria occidentalis, Teramnus labialis, Terminalia bellirica, Terminalia chebula, Tetraclinis articulata Benth., Tetrapleura tetraptera, Teucrium polium, Thunbergia laurifolia Linn, Tinospora cordifolia, Tinospora crispa, Tournefortia hirsutissima, Tragia involucrata, Tribulus terrestris, Trichosanthes cucumerina, Trichosanthes kirilowii, Tricosanthes anguina, Trigonella foenum-graecum, Triticum vulgare , Turnera diffusa, ursolic acid, Vaccinum myrtillus , Valeriana officinalis , Vanadium, Vernonia amygdalina, Viburnum foetens, Vinca rosea, Viscum album, vitamin B (1,2,5,6,12), vitamin C, Wedelia paludosa, Withania somnifera , Xanthium strumarium , Xanthocercis zambesiaca , yacou root, Zingiber officinale, Zizyphus sativa, Zizyphus spina-christi, Zygophyllum coccineum and Zygophyllum gaetulum.

In a further preferred embodiment, the compositions also include a chromium compound. While not intending to be limited by theory, various studies have indicated that chromium supplementing will improve glucose tolerance in insulin-sensitive individuals by up to 50% and thereby maximize insulin efficiency.

Chromium is a constituent of a biologically active compound, the glucose tolerance factor (GTF), found in foods such as organ meats, whole grains, cheese, mushrooms and brewer's yeast. Various chromium compounds may be included in the compositions, and in amounts effective to improve insulin efficiency. A preferred chromium compound is chromium picolinate, which may be included, for example in an amount of from about 50 to about 500 micrograms per 100 grams of supplement.

Lipoic acid (also known as alpha-lipoic acid, thioctic acid or 6,8-dithio octanoic acid) is a nutrient that the human body makes in minute quantities and may be obtained from yeast and liver. Studies have shown that lipoic acid can significantly increase the body's utilization of blood sugar in type II diabetics and that lipoic acid may increase the metabolic clearance rate of glucose by 50% in diabetics. In Europe, lipoic acid has been used as a substitute for insulin in the treatment of Type II diabetes.

Thus, while not necessary for achieving the effects of the present invention, these additional compounds may be added to the present compositions for enhancing their effect.

As shown in the Examples section, the *Morus* leaves extract (either powder or liquid) was successfully mixed with various parts of a variety of plants (seeds, bark, leaves etc.) and in various forms thereof (for example the herb, extract or tincture).

The compositions described herein may further comprise at least one carrier.

The carrier may be a liquid carrier (such as water, alcohols, saline, oil and juice) or a solid carrier (such as maltodextrin, dextrins, silicon dioxide, starches, gums and hydrocolloids).

Examples of hydrocolloids include absorbable collagen, polylactic acid polymer (OPLA), calcium sulfate, tricalciumphosphate (TCP), hydroxyapatite (HA), biphasic TCP/HA ceramic, polylactic acids and Polyanhydrides.

For example, the compositions of the invention were successfully encapsulated, such that each capsule contained 500 mg of which 40% by weight are herbal extracts and 60% by weight were the TCP carrier. This compositon was termed hereinafter "Sugar Red" capsule formulation.

In choosing the suitable carrier, care should be taken to choose a carrier which is suitable for the treatment of diabetic patients.

The compositions of the invention may be in the form of a tea, a tincture, a concoction, an infusion, a tablet, a capsule, a pill, a bar, a sachet, a lozenge, a pastille, a chewable gum, a lotion, a powder or granules. Accordingly, a carrier suitable of each of these forms is chosen.

The term "Tea" is inclusive of a number of herb extracts and powders, capable of being dispersed and/or dissolved upon contact with water. This term also includes "instant teas" which appear as powdery, granular or paste-like precursors. Used for tea preparation.

In a preferred embodiment, the active compounds or plant species added to the *Morus* extract are used in the form of a tincture. As used herein, the term "tincture" means an alcoholic extract of the herb, or a solution of the active compounds of the plant species in an alcoholic solvent.

In another preferred embodiment, the compositions of the invention appear in the form of a decoction. As used herein, the term "decoction" means a water extract of bark or roots prepared at a low boil for 10-20 minutes.

The "Infusion" form is similar to a "decoction" form, but is made by steeping plants or plant extracts in hot water, rather than in boiling water, for 10-20 minutes.

The term "tablet" refers to a pharmacological composition in the form of a small, essentially solid pellet of any shape (cylindrical, spherical, rectangular, capsular or irregular) and is intended to embrace compressed tablets, coated tablets, matrix tablets, osmotic tablets, and other forms known in the art.

The term "capsule" is intended to embrace capsules in which the body of the capsule disintegrates after ingestion to release particulate contents which exhibit the desired sustained-release behavior, and also capsules for which the body of the capsule remains substantially intact during its residence in the GI tract. Capsules are prepared by loading a powdered composition into a capsule, optionally together with an inert carrier. Preferred are capsules of the invention are made from gelatin.

The term "pill" is used interchangeably with the terms "tablet" or "capsule".

The term "sachet" is used to denote a relatively small bag or envelope-like packet, for example as in a tea bag.

The term "lozenge" can refer to any solid or semi-solid substrate wherein at least a majority of the substrate is designed to dissolve in an oral cavity. The term "pastille" refers to a subclass of lozenges; that is, molded lozenges.

The term "bar" refers to a block of solid substance that is chewable or edible.

The term "chewable gum" is intended to mean a composition which comprises substantially water-insoluble, chewable plastic gum base such as chicle, or substitutes thereof, including jelutong, guttakay rubber or certain comestible natural or synthetic resins or waxes. Incorporated with the gum base in admixture therewith may be plasticizers or softening agents, e.g., glycerine, and a flavoring composition which incorporates one or more of the compositions of the present invention, and in addition artificial sweeteners such as cyclamates or saccharin. Other optional ingredients may also be present.

The term "lotion" has been used to categorize many topical suspensions.

The term "powder" refers to a particulate material consisting of a loose aggregation of finely divided solid particles. For a fine powder the maximum dimension is smaller than 1 millimeter and the average particle size is less than 100 microns.

The term "granule" refers to a bead which has been dried to a moisture content below about 15%.

The present invention also provides for the use of the above combination of plant parts/extracts in the preparation of a food supplement or a pharmaceutical composition.

Thus, according to a preferred embodiment of the present invention, there is provided a dietary composition which comprises the herbal compositions described herein and a dietetically acceptable excipient.

Preferably, the compositions of the present invention can be used as a dietary supplement and/or form a part of a food product.

The term "dietary supplement" as used hereinabove and hereinbelow includes a composition which may be used without prescription by a third party, for example, a physician. The components may be taken together with meals or separated thereof, on a daily basis or only sometimes.

The term "food product" refers to material of either plant or animal origin, or of synthetic sources, that contain an essential body nutrient such as a carbohydrate, protein, fat, vitamin, mineral, etc. Examples include meats, fruits, vegetables, grains, nuts, and the like.

According to another preferred embodiment of the present invention, there is provided a pharmaceutical composition which comprises the herbal compositions described herein and a pharmaceutically acceptable excipient.

Pharmaceutically acceptable excipients are any materials that do not interfere with the pharmacological activity of third composition or degrade the bodily functions of the subject to which it can be administered, but facilitate fabrication of dosage forms or actual administration of the composition; for example by improving palatability of oral dosage forms. Examples of pharmaceutically acceptable excipient include but are not limited to maltodextrin, calcium phosphate, and fused silica. Pharmaceutically and dietarically acceptable excipients also include flavorants, as well as various additives such as other vitamins and minerals, all solvents, dispersion media, coatings, isotonic and absorption delaying agents, sweeteners and the like, non-toxic auxiliary substances such as wetting or emulsifying agents, pH buffering agents and the like, such as for example, sodium acetate, sorbitan monolaurate, triethanolamine oleate, and inert ingredients such as talc and magnesium stearate which are standard excipients in the manufacture of tablets, capsules and other dosage forms.

As shown in the examples section which follows, the compositions of the present invention were successfully used to treat diabetes, conditions associated therewith and dyslipidemia.

Thus, according to a preferred embodiment of the present invention, the compositions of the present invention are used in the preparation of a medicament for treating and/or preventing diabetes and/or conditions associated therewith. According to another embodiment, the medicament is for treating and/or preventing dyslipidemia.

Therefore, the present invention also encompasses any of the compositions described herein, being packaged in a packaging material and identified in print, in or on said packaging material, for use in the treatment and/or prevention of diabetes and/or conditions associated therewith or for use in the treatment and/or prevention of dyslipidemia.

According to another aspect of the invention, there is also provided a use of the compositions described herein in the preparation of a medicament for treating and/or preventing diabetes and/or conditions associated therewith.

As used herein, the terms "anti-diabetic" or "hypoglycemic" compound, composition or medicament, generally refers to an agent that lowers blood glucose levels.

As shown in the Examples section which follows, the efficiency of the present inventions as anti-diabetic agents, was demonstrated by the significant drop in the measured HbAlC factor (as defined hereinabove) after a three months treatment regime.

As a general guideline, and without being limited to the values suggested herein, if a diabetic patient blood HbAIC (AlC) levels are decreased by at least 0.5%, then the compound is considered to be a hypoglycemic agent. Such an agent or any other agent that may lower blood glucose levels to other accepted standards of hypoglycemic effect, may be used to treat diabetes or to prevent the incidence of diabetes.

According to yet another aspect of the invention, there is also provided a use of the compositions described herein in the preparation of a medicament for treating and/or preventing dyslipidemia.

Preferably, the compositions provided by the present invention are used as an orally-administrable composition. The term "orally-administrable composition" as used herein includes both pharmaceutical and herbicidal compositions, as well as food supplements within its scope. However, other forms of administration are also possible, as disclosed hereinunder.

According to an additional aspect of the invention, there is provided a method of treating and/or preventing diabetes and/or conditions associated therewith, the method comprising administering to a subject in need thereof a therapeutically effective amount of any of the compositions described herein.

As exemplified hereinbelow, the present compositions successfully treated type II diabetes and conditions associated therewith. Examples of type II diabetes associated conditions include atherosclerosis, hypertension, diabetic retinopathy, diabetic nephropathy, diabetic polyneuropathies, thyroid disorders, leg ulcers, diabetic foot, liver diseases, kidney function, sight, impotence and constipation.

According to an additional aspect of the invention, there is provided a method of treating and/or preventing dyslipidemia and/or conditions associated therewith, the method comprising administering to a subject in need thereof a therapeutically effective amount of any of the compositions described herein.

By the term "administering," it is meant that the composition is delivered to a subject by any means or route which is effective to achieve the desired result, including, e.g., oral, parenteral, enteral, intraperitoneal, topical, transdermal, ophthalmic, nasally, local, non-oral, such as aerosal, inhalation, subcutaneous, intravenous, intramuscular, buccal, sublingual, rectal, vaginal, intra-arterial and intrathecal. Oral administration is especially preferred.

The compositions of the present invention as disclosed hereinabove and exemplified hereinbelow may be prepared and delivered in a number of different forms in order to allow the forementioned modes of administration. They can be administered alone, or in combination with other active or inert agent(s). The composition of the invention is administered to a subject in a therapeutically-effective amount, that is, an amount that will provide a concentration of the herbal extracts that is capable of exerting the desired therapeutic effect. It has been found, in general terms, that the compositions of the present invention need to be administered in amounts such that, typically, a daily dose for an adult contains 1000 mg and 6000 mg (dry weight) of the herbal combination, more preferably between 1500 mg per day to about 3 grams per day, the precise values depending on the particular combination of extracts used, and on the mode of delivery.

As used herein, a "dose" or "dosage" refers to a specified quantity of a therapeutic agent prescribed to be taken at one time or at stated intervals. Thus, the total daily amount prescribed (daily dose) may be administered in a single dose or in more than one dose which may be taken at different times throughout the day.

It will be readily apparent that all of the above compositions in their alternate forms may be used alone or in combination to provide an anti-diabetic or anti dyslipidemia herbal medicine, which when administered to a patient, results in diabetes or dyslipidemia preventive or therapeutic effect.

Additional objects, advantages, and novel features of the present invention will become apparent to one ordinarily skilled in the art upon examination of the following examples, which are not intended to be limiting. Additionally, each of the various embodiments and aspects of the present invention as delineated herein and as claimed in the claims section below finds experimental support in the following examples.

### EXAMPLES

Reference is now made to the following examples, which together with the above descriptions, illustrate the invention in a non limiting fashion.

### MATERIALS AND ANALYTICAL METHODS

### Materials:

Fresh, young mulberry leaves (*Morus alba* and *Morus nigra*) were collected from a wild population procured in bulk from Regional Judea district mainly.

Unless otherwise specified, all herbs were obtained from Tamar Medical Ltd., Israel. The extracts were also commercially available from other various sources: Naturex (Avignon, France); Blue California (California, USA); PLThomas (New Jersey, USA); Draco Natural Products (California, USA); Watson Industries (California, USA); Sun Ten Pharmaceutical Co. (Taipei, Taiwan); Integrity (Florida, USA), Sabinsa (NJ, USA).

Chromium Picolinate and alpha Lipoic Acid were obtained from VITALIFE.

Tricalciumphosphate and gelatine capsules (size 0) were obtained from Karmat Micro-Encapsulation, Kibbutz Ramot Menashe.

Prepared capsules were kept in safety-closed plastic bottles with labels, each bottle containing 90 capsules, and stored in a cool dry place away from direct sunlight or moisture.

### Instrumental Data:

Mixing was conducted using large food processors.

Grinding was conducted using a wet grinding machine STEPHAN electric grind processor.

Pressing of leaves was done using a screw presser.

Spray drying was conducted using a fluid bed coater (Wurster process) -CPI.

Blood cholesterol and other venous blood samples were collected and measured using HMO Laboratories medical staff and instruments.

### EXAMPLE 1

### Preparation of Morus liquid and dry extracts

*Morus alba* leaves (25 Kg) and *Morus nigra* leaves (25 Kg) were thoroughly washed with running tap water (20 liters), strained and grained using a wet grinding mill. The ground leaves were left standing for about 30 minutes for latex to be exuded from the cut leaves and only then were they pressed until juice was collected. The obtained juice was concentrated into a fluid extract by evaporation and was stored in a cool place. The squeezed leaves were separately collected into sacks which were brewed together with the concentrate. The effects of brewing time on alpha-glucosidase inhibitory active component were studied, showing significant differences between the different samples. The most effective inhibition was observed when 8 to 10 minutes brewing time was applied but up to 20 minutes of brewing did not adversely affect the properties of the extract.

Following brewing, the mixture was rapidly chilled. The obtained solution was filtered and was used as a solution (hereby termed liquid *Morus* extract or *Morus* juice) or was spray dried into a powder mash by a fluid bed coater at lower temperature then 40°C, sieving the product through a 600 microns mesh, to obtain a dry *Morus* extract (also termed *Morus* powder).

### EXAMPLE 2

### Preparation of capsules containing Morus, Urtica , Radix and Artemisia

*Urtica Dioica* dried herb (100 grams), *Taraxacum Officinale* (Dandelion) dried root (120 grams) and *Artemisia Judaica* dried herb (120 grams) were ground into fine powder (600 microns mesh) and were mixed with the *Morus* powder (660 grams) prepared according to Example 1. Part of the resulting mixture (500 mg) was loaded into capsules. The capsule composition is described in Table 1 below:

**Table 1**

| Plant | Weight Percent |
|---|---|
| *Morus* Folium (*Morus alba* and *nigra*) pressed Juice dry extract powder | 66 |
| *Urtica Dioica* herb | 10 |
| *Artemisia Judaica* herb | 12 |
| *Taraxacum Officinale* Root | 12 |

### EXAMPLE 3

### Preparation of capsules containing Morus, Urtica, Radix, Artemisia and Canella

The process of Example 2 was repeated, further grinding cinnamon bark herb along with the *Urtica Dioica* dried herb, the *Taraxacum Officinale* dried root and the *Artemisia Judaica* dried herb, and the resulting mixture was loaded into capsules. The capsule composition is described in Table 2 below:

**Table 2**

| Plant | Weight percent |
|---|---|
| *Morus* Folium (*Morus alba* and *nigra*) pressed Juice dry extract powder | 64 |
| *Urtica Dioica* herb | 10 |
| *Artemisia Judaica* herb | 8 |
| *Taraxacum Officinale* (Dandelion) Root | 10 |
| *Canella winteriana* (Cinnamon bark) | 8 |

### EXAMPLE 4

### Preparation of capsules containing Morus, Urtica, Radix, Artemisia, Canella and Humulus

The process of Example 3 was repeated, further grinding *Humulus Lupulus* dry strobiles along with the *Urtica Dioica* dried herb, the *Taraxacum Officinale* dried root, the *Artemisia Judaica* dried herb and the cinnamon bark herb, and the resulting mixture was loaded into capsules. The capsule composition is described in Table 3:

**Table 3**

| Plant | Weight percent |
|---|---|
| *Morus* Folium (*Morus alba* and *nigra*) pressed Juice dry extract powder | 60 |
| *Urtica Dioica* herba | 8 |
| *Artemisia Judaica* | 6 |
| *Taraxacum Officinale* (Dandelion) Root | 8 |
| *Canella winteriana* (Cinnamon bark) | 8 |
| *Humulus Lupulus* dry strobiles | 10 |

### EXAMPLE 5

### Preparation of capsules containing Morus, Urtica extract, Artemisia and Gymnema extract

*Artemisia Dracunculus* dried herb was grinded into fine powder and was mixed with the *Morus* extract powder prepared as described in Example 1, and with the *Gymnema* and *Urtica* standardized extracts. The herb mixture was placed in capsules. The capsule composition is described in Table 4 below:

**Table 4**

| Plant | Weight percent |
|---|---|
| *Morus* Folium (*Morus alba* and *nigra*) pressed Juice dry extract powder | 60 |
| *Urtica Dioica* standardized extract (0.8% beta sitosterol) | 15 |
| *Artemisia Dracunculus* (Tarragon) herb | 10 |
| *Gymnema Sylvestre* standardized extract (75% Gymnemic Acid) | 7 |

### EXAMPLE 6

### Preparation of capsules containing Morus, Artemisia, Urtica extract, Radix extract, Cinammon extract and Trigonella extract

*Artemisia Alba* dried herb was grinded into fine powder and was mixed with the *Morus* extract powder prepared as described in Example 1, and with the *Urtica* dioica, *Radix taraxacum Cinnamon* cassia and *Trigone*/*la foenum graecum* standardized extracts. The herb mixture was placed in capsules. The capsule composition is described in Table 5 below:

**Table 5**

| Plant | Weight percent |
|---|---|
| *Morus* Folium (*Morus alba* and *nigra*) pressed Juice dry extract powder | 30 |
| *Urtica Dioica* standardized extract (0.8% beta sitosterol) | 8 |
| *Artemisia Alba* herb | 8 |
| Dandelion Standardized extract (20% glycosaponins) | 8 |
| Cinnamon cassia standardized extract (3% trimeric & tetrameric Type A polymers) | 6 |
| *Trigonella foenum graecum* (Fenugreek) standardized extract (80% saponins) | 20 |

### EXAMPLE 7

### Preparation of capsules containing Morus, Artemisia and Urtica extract

*Artemisia Alba* dried herb was grinded into fine powder and was mixed with the *Morus* extract powder prepared as described in Example 1, and with the *Urtica* standardized extract. The herb mixture was placed in capsules. The capsule composition is described in Table 6 below:

**Table 6**

| Plant | Weight percent |
|---|---|
| *Morus* Folium (*Morus alba* and *nigra*) dry extract powder | 80 |
| *Urtica Dioica* standardized extract (0.8% beta sitosterol) | 10 |
| *Artemisia Alba* herb | 10 |

### EXAMPLE 8

### Preparation of capsules containing Morus, Artemisia and Urtica extract

*Artemisia pallens Wall* dried herb was grinded into fine powder and was mixed with the *Morus* extract powder prepared as described in Example 1, and with the *Urtica* standardized extract. The herb mixture was placed in 500 mg capsules. The capsule composition is described in Table 7 below:

**Table 7**

| Plant | Weight percent |
|---|---|
| *Morus* Folium (*Morus alba* and *nigra*) dry extract powder | 85 |
| *Urtica Dioica* standardized extract (0.8% beta sitosterol) | 7.5 |
| *Artemisia pallens Wall* | 7.5 |

### EXAMPLE 9

### Preparation of capsules containing Morus, Urtica extract and Lagerstroemia extract

*Artemisia Alba* dried herb was grinded into fine powder and was mixed with the *Morus* extract powder prepared as described in Example 1, and with the *Urtica* and banaba (*Lagerstroemia speciosa*) standardized extract. The herb mixture was placed in 500 mg capsules. The capsule composition is described in Table 8 below:

**Table 8**

| Plant | Weight percent |
|---|---|
| *Morus* Folium (*Morus alba* and *nigra*) pressed Juice dry extract powder | 60 |
| *Urtica Dioica* standardized extract (0.8% beta sitosterol) | 7.5 |
| *Artemisia Alba* herb | 7.5 |
| *Lagerstroemia speciosa* (Banana) standardized extract (60% corosolic acid) | 25 |

### EXAMPLE 10

### Preparation of capsules containing Morus, Artemisia, Urtica extract and chromium picolinate and alpha lipoic acid

*Artemisia Alba* dried herb was grinded into fine powder and was mixed with the *Morus* extract powder prepared as described in Example 1, and with the *Urtica* standardized extract, as well as with chromium picolinate and alpha lipoic acid. The herb mixture was placed in 500 mg capsules. The capsule composition is described in Table 9 below:

**Table 9**

| Plant | Weight percent |
|---|---|
| *Morus* Folium (*Morus alba* and *nigra*) dry extract powder | 65 |
| *Urtica Dioica* standardized extract (0.8% beta sitosterol) | 10 |
| *Artemisia Alba* herba | 10 |
| Chromium Picolinate | 0.01 (100 mg) |
| Alpha Lipoic Acid | 15 |

### EXAMPLE 11

### Preparation of a Decoction containing Morus, Urtica, Artemisia, Cannella and Radix

*Artemisia alba, Urtica dioica, Canella winteriana* and *Taraxacum Officinale* dried herbs was grinded into fine powder and was mixed with the *Morus* extract powder prepared as described in Example 1. A decoction was prepared from the obtained mixture by adding 500 grams of water. The liquid was strained and stored in a cool place. The mixture composition, before the addition of water, is described in Table 10 below:

**Table 10**

| Plant | Weight Percent |
|---|---|
| *Morus* Folium (*Morus alba* and *nigra*) dry extract powder | 60 |
| *Urtica dioica* herb | 5 |
| *Artemisia judaica* herb | 5 |
| *Canella winteriana* herb | 15 |
| *Taraxacum Officinale* dried root | 15 |

### EXAMPLE 12

### Preparation of Herb Tincture Containing Morus, Urtica, Artemisia, Cannella and Radix

The *Artemisia Judaica* and *Urtica dioica* herb tinctures were mixed with the *Morus* extract pressed juice prepared as described in Example 1 and were stored in a cool place. The obtained tincture was stable in refrigeration for about 4 weeks. The tincture composition is described in Table 11 below:

**Table 11**

| Plant | Weight percent |
|---|---|
| *Morus* Folium (*Morus alba* and *nigra*) pressed juice | 80 |
| *Urtica dioica* herb tincture | 12 |
| *Artemisia Judaica* tincture | 8 |

### EXAMPLE 13

### Preparation of Herb Infusion Containing Morus, Artemisia, Urtica, Punica, Salix and Olea

The *Artemisia Judaica* herb (1600 grams), *Urtica dioica* herb (2400 grams), *Punica Granatum seed oil (2000 ml), Salix alba* leaves (600 grams) and *Olea Europea* leaves (600 grams) were mixed with the fresh *Morus* extract pressed juice (12800 ml) prepared as described in Example 1 and the mixture ass heated to 40 °C for 20 minutes. The mixture was allowed to cool and was then filtered and stored in a cool place. A clear greenish-brown infusion was obtained (13000 ml). Its composition is described in Table 12 below:

**Table 12**

| Plant | Weight percent |
|---|---|
| *Morus* Folium (*Morus alba* and *nigra*) pressed juice | 64 |
| *Urtica dioica* herb | 12 |
| *Artemisia Judaica herb* | 8 |
| *Punica Granatum* seed oil | 10 |
| *Salix alba bark* | 3 |
| *Olea Europea leaves* | 3 |

### EXAMPLE 14A

### Preparation of "Sugar Red" liquid formulations and capsules

*Morus alba & nigra fresh pressed juice, Taraxacum officinale, Urtica dioica, Artemisia arborescens, Cinnamomum cassia* and *Rosa canina* were combined to produce the "Sugar Red" (SR) liquid formulation, as follows:
*Morus* leaves (200 Kg) were processed as described in Example 1, to obtain pressed *Morus* juice (50 Kg). The pressed juiced was transferred into a double walled tank and water (50 Kg) were added. The squeezed leaves left after the initial pressing stage were collected, put into sacks, and added to the tank. Urtica dried herb (0.5 Kg), Dandelion dried root (0.4 Kg), Artemisia dried herb (0.4 Kg), cinnamon bark (0.4 Kg) and Rosa Canina (0.3 Kg) were also put into sacks and added to the tank. The mixture in the tank was brewed for 10 minutes, left to cool to room temperature and filtered yielding liquid Sugar Red formulation (100 Kg).

### EXAMPLE 14B

### Preparation of "Sugar Red" capsules

*Morus* leaves (400 Kg) were processed as described in Example 1, to obtain pressed *Morus* juice (100 Kg). The pressed juiced was transferred into a double walled tank. The squeezed leaves left after the initial pressing stage were collected, put into sacks and added to the tank, finally adding water thereto (100 Kg). The mixture in the tank was brewed for 10 minutes, left to cool to room temperature and filtered. *Morus* pressed juiced was then concentrated into a fluid extract (50 Kg), which was spray dried in the presence of the carrier tricalciumphosphate (TCP) using a fluid bed coater according to Wurster process-CPI comp., to obtain *Morus* powder (7 Kg) of which 40 % by weight was the *Morus* herbal extract and 60 % by weight was the TCP carrier. Urtica dried herb (1 Kg), Dandelion dried root (0.8 Kg), Artemisia dried herb (0.8 Kg), cinnamon bark (0.8 Kg) and Rosa Canina (0.6 Kg) were grained into fine powder and were mixed with the *Morus* powder (6 Kg). Gelatin Capsules size 0 were prepared from the mixture yielding 200,000 capsules, such that each capsule contained 500 mg.

### EXAMPLE 15

### Treating a diabetic human subject with the herb mixture of Example 13 (Infusion)

Patients were given three doses of 150 ml infusion each day, taken by drinking, as follows:
a) The treated subject was a 75-year-old man having diabetes for 20 years, of which the last 8 years were on insulin treatment, s/p M.I,(myocardial infarction) LICA (left inferior coronary artery) stenosis, congestive hurt failure, peripheral vascular disease, hypertensive, hyperlipidemia, and chronic renal insufficiency. He was presented with a weight of 64 kg, and a body mass index of 22. He was on insulin, furosemide (eg, Lasix) 120 mg daily, mononit 40 mg twice a day, norvasec 5mg daily and bezalip 40mg daily. His initial fasting blood sugar levels while using the insulin ranged from 150-250%. A total cholesterol test result was 214%, triglyceride 140mg%, creatinine 2.67mg%, urea 67 mg% and an electrocardiogram revealed atrial fibrillation with a ventricular rate of 76/minute mild retinal changes. Following a 3 months treatment with a daily dose of 3 x 150 ml mixture according to example no 13, the subject's fasting blood sugar level fluctuated between 150+/-30 mg%, and insulin was stopped. A progressive fall in his fasting blood sugar level to 130+/-20 mg% was observed. During this time, his hemoglobin HbA1C came down from 11.1 to 7.2 and was steadily improving.
b) In another diabetic patient, the treated subject was a 54 -year-old man, height: 164cm; weight: 73.5kg BMI: 27.3 Hypertensive, diabetes since 1999. Blood glucose , 290 mg/dl, cholesterol (TC) - 282 mg/dl, triglycerides -616 mg/dl Urine: glucose-U strip 1000, protein negative. Diagnosed as Diabetes II, had a very strict diet but after a short while he had to take oral hypoglycemics: metformin and glyburide one tablet of each three times a day were prescribed. Instructed to maintain low sugar low fat diet and exercise, he reduced weight and at his first appointment was 62 kg BMI: 23.1. Fasting blood sugar levels fluctuated 250+/-50 mg/dl, HAB1c was 9.8. And still Dyslipidemia, renal function was normal. This subject began treatment with 150 ml herbal preparation according to example No 13 three times a day, and continued oral hypoglycemic for another 4 weeks, whereas at this period drugs were reduced rapidly. The subject stopped taking oral hypoglycemic drugs and his Fasting blood glucose was constant 100+/- 10 mg/dl.
c) In yet another diabetic patient, the treated subject was a 70 year old patient with diabetes mellitus for 30 years, maintained on combined oral hypoglycaemics: [Gluben (glibenclamide) + glucophage + prandase] X 3 times a day. The patient gradually developed retinopathy and microanurisms and edema were observed mainly in the left eye, localized in the vicinity of the macula. Focal Laser treatment was performed above the left fovea. Because control of diabetes remained poor prior to the first appointment (with HbA1c 11) insulin treatment was suggested. C-peptide levels in the low range - 336 pmol/L (364-1655). The patient began treatment with 150 ml herbal preparation according to example No 13 three times a day, was very cooperative, continued oral, hypoglycemic for another 6 weeks as was prescribed by his family doctor, at this period drugs were reduced according blood glucose levels, and after 16 weeks he was asked to stop glucophage + prandase (ascarbose). Fasting blood glucose results were still labile 150+/- 40 mg/dl. After six months patient was reassessed by his ophthalmologist which found "*No Diabetic Retinopathy*"*,* repeated result were constant.

### EXAMPLE 16

### Treating a Diabetic Human Subject with the Herb Mixture of Example 12 (Tincture)

The treated subject was a 55-year-old woman with a 23-year history of type 2 diabetes (initiated as gestational diabetes) complicated by proliferative retinopathy (1955), left eye cataract (operated), and right vitrectomy later on, intermittently elevated urinary albumin excretion rates, peripheral polyneuropathy. Vascular insufficiency, tibial vein trombectomy, proximal anastomosis jamp to posterior tibial artery. Rheumatoid arthritis. Her diabetes was treated with subcutaneous insulin for the last ten years. Her medications included insulin, convertin 5mg daily, cumadin 2.5mg daily and lipitor (atrovastatin) 10 mg daily. Initially her management was to continue the insulin for 6 weeks, adding 30 ml X 3/day herbal preparation according to example No 12. Then, insulin was gradually reduced, and metformin and glibetic (glibenclamide) were initiated trice daily each. Blood glucose was significantly reduced 180+/- 20 to 130+/- 20 mg%. HbA1C came down from 10.6 to 6.27 and was steadily improving.

### EXAMPLE 17

### Treating a diabetic human subject with the herb mixture of Example 11 (decoction)

The treated subject was a 56-year-old patient. Height: 166 cm; weight: 67kg. BMI: 24.3 Presented with a history of treated Diabetes type 2 for 6 years, hypertension, hyperuricemia, and dyslipidemia, blood glucose 350+/-50, HBA1c - 10.69, total cholesterol of 262 mg/dl and triglyceride - 316 mg/dl, uric acid - 4.6 mg/dl. Hypertension was managed with herbal remedies, hyperuricemia -zylol 30 mg daily, diabetes was managed with glyburid and prandase (acarbose) tid each, including the regimen of strict diet and exercise.

The patient began treatment with 150 ml herbal preparation according to example No 11 three times a day. Patient continued oral, hypoglycemic for another 3 weeks as was prescribed by his care giver, at this period drugs were reduced rapidly monitoring his blood glucose levels, and after 5 weeks he was asked to stop oral hypoglycemic. Fasting blood glucose was constant 100+/- 20 mg/dl.

### EXAMPLE 18

### Treating diabetic human subjects with the herb mixture of Example 3 (Capsules)

Patients 1-4, all suffering from diabetes type II, were treated three times a day with 2 X 500 mg capsules of the herbal composition prepared according to Example 3. Blood cholesterol and HbA1C were recorded before treatment and after 3 months treatment with herbal composition (example 3). Results are presented in table 13 below:

**Table 13**

| Patient | year born | Cholesterol | | HbA1C | |
|---|---|---|---|---|---|
| | | pretreatment | post treatment | pretreatment | post treatment |
| 1 | 1954 | 199 | 144 | 7.3 | 6 |
| 2 | 1952 | 165 | 139 | 8.3 | 6.1 |
| 3 | 1953 | 230 | 137 | 10.7 | 9.5 |
| 4 | 1954 | 175 | 144 | 8.1 | 7.6 |

### EXAMPLE 19

### Clinical Trials of diabetic human subjects using the liquid Sugar Red formation of Example 14A

Clinical evidence of 26 diabetic human subjects using the liquid herb mixture of Example 14A (Sugar Red liquid form) who have been suffering for many years and were treated with conventional medicines, is presented herein. Prior to treatment with the formulation of Example 14A, patients have been suffering from various levels of the disease and had customary medical complaints associated with NIDDM, blood fats, and functional damages of the cardio-vascular system, kidneys, liver and various problems as impotence. All were under the supervision of specialized centers including continues assessment by cardiologists, cardiovascular surgeons or ophthalmologists. Fasting blood sugar, HBA1c, CBC, renal and liver functions, cholesterol, triglyceride, height and weight were measured at baseline (visit 1) and served as the basis for the effectiveness of evaluation of Sugar-Red treatment.

150 ml of the liquid Sugar-Red formulation were given 3 times a day, right after glucose monitoring, and half an hour before each metal. During the first month, patients weekly visited the clinic for a check- up. Change in medications was instructed according to blood glucose monitoring. While every 3-4 months total blood screening was performed at the diabetic centers the patients were treated.

The results shown in Figure 1 show that fasting blood glucose level dropped by 44.2% ± 16% after three months of treatment (n= 26 patients).

Similarly, the results shown in Figure 2 show that HbA1C levels dropped by 33.94% ± 12.66% after three months of treatment (n= 22 patients).

### EXAMPLE 20

### Double blind study of diabetic human subjects using the herb mixture of Example 14B (Sugar Red Capsules)

Clinical evidence of 8 diabetic human subjects using the herb mixture of Example 12 (Sugar capsules) who have been suffering for many years and were treated with conventional medicines is presented herein. Prior to treatment with the formulation of Example 14B, patients have been suffering from various levels of the disease and had customary medical complaints associated with NIDDM, blood fats, and functional damages of the cardio-vascular system, kidneys, liver and various problems as impotence. All were under the supervision of specialized centers including continues assessment by cardiologists, cardiovascular surgeons or ophthalmologists. Fasting blood sugar, HBA1c, CBC, renal and liver functions, cholesterol, triglyceride, height and weight were measured at baseline (visit 1) and served as the basis for the effectiveness of evaluation of Sugar-Red treatment.

Two Sugar-Red capsules (500 mg each) were given 3 times a day, right after glucose monitoring, and half an hour before each meal. During the first month, patients weekly visited the clinic for a check- up. Change in medications was instructed according to blood glucose monitoring. While every 3-4 months total blood screening was performed at the diabetic centers the patients were treated. The efficacy results for 8 diabetic patients are shown in Table 14 below:

**TABLE 14**

| Patient Code | drug regiment | | Age (Year born) | CHOLESTEROL | | HbA1C | | |
|---|---|---|---|---|---|---|---|---|
| | **Pre Sugar Red treatment** | **Post Sugar Red treatment** | | **Chol1** | **Chol2** | **Hb1** | **Hb2** | **Hb3** |
| ALB | insulin | No insulin Gastro | 1951 | 154 | 118 | 9.9 | 8.1 | 10.6 |
| DAM | No drugs | | 1963 | 185 | | 8.5 | 7.3 | |
| DAL | Glucoph x3 | No drugs | 1954 | 199 | 144 | 7.3 | 6 | |
| AMO | Glucoph x3 | No drugs | 1952 | 165 | 139 | 8.3 | 6.1 | |
| EST | Avad, Novo(x3) Gluco,(X3) Simo | Novo,(X1) Gluco,(x2) Simo, SR | 1954 | 175 | 144 | 8.1 | 7.6 | 7.32 |
| AVI | Insulin | No Insulin, Gluco,(x2) | 1952 | 149 | | 8.1 | 7.7 | |
| RAY | Ayad, Novo,x3 Glucox3 | Gluben,x1 Gluco, x2, SR | 1926 | 172 | | 8.91 | 7.29 | 7.48/7.35 |
| BEN | Novo | Novo | 1953 | 230 | 137 | 10.7 | 9.5 | |

| | | | | | | | | |
|---|---|---|---|---|---|---|---|---|
| * Simovil-simvastatin=simo, Glucophage=gluco, Glubenclamide -gluben, Novonorm-repaglinide=Novo | | | | | | | | |

## Claims

1. A herbal composition comprising:
at least one *Urtica* species or an extract thereof,
at least one *Artemisia* species or an extract thereof, and
an extract of at least one *Morus* species,
wherein said extract of a *Morus* species is prepared of *Morus* leaves and
comprises *Morus* latex.

2. The composition according to claim 1, wherein said *Morus* species is selected from the group consisting of *Morus alba, Morus bombycis, Morus indica, Morus insignis, Morus nigra* and *Morus Australis,* and any combinations thereof, and/or wherein said *Urtica* species is selected from the group consisting of *Urtica dioica, Urtica urens* and *Urtica piluflfera,* and any combinations thereof, and/or wherein said *Artemisia* species is selected from the group consisting of *Artemisia dracunculus, Artemisia herba alba, Artemisia pallens Wall, Artemisia roxburghiana* and *Artemisia Judaica,* and any combinations thereof.

3. The composition of claim 1, wherein an amount of said *Morus* extract ranges from about 50 weight percentage to about 90 weight percentages of the total weight of the composition, an amount of said *Artemisia* species ranges from about 1 weight percentage to about 20 weight percentages of the total weight of the composition, and an amount of said *Urtica* species ranges from about 2 weight percentage to about 20 weight percentages of the total weight of the composition.

4. The composition according to claim 1, which further comprises at least one species selected from a *Cinnamomum* species, a *Canella* species, a *Taraxacum* species and/or a *Rosa* species.

5. The composition according to claim 4, wherein said *Cinnamomum* species is selected from the group consisting of *Cinnamomum cassia, Cinnamomum zeylanicum; Cinnamomum saigonicum, Cinnamomum aromaticum* and *Cinnamomum laurus,* and/or wherein said *Canella* species is *Canella winterana,* and/or wherein said *Taraxacum* species is *Taraxacum Officinale,* and/or wherein said *Rosa* species is *Rosa canina.*

6. The composition of claim 4, wherein an amount of said *Morus* extract ranges from about 50 weight percentage to about 90 weight percentages of the total weight of the composition, an amount of said *Artemisia* species ranges from about 1 weight percentage to about 20 weight percentages of the total weight of the composition, an amount of said *Urtica* species ranges from about 2 weight percentage to about 20 weight percentages of the total weight of the composition, and an amount of any species selected from a *Cinnamomum* species, a Canella species, a *Taraxacum* species and/or a *Rosa* species ranges from about 5 weight percentages to about 30 weight percentages of the total weight of the composition.

7. The composition of any of claims 1-6, further comprising one or more species selected from a *Humulus* species, a *Gymnema* species, a *Trigonella* species, a *Punica* species, a *Salix* species, and/or an *Olea* species.

8. The composition of any of claims 1-7, further comprising at least one carrier.

9. The composition of any of claims 1-8 being in the form of a tea, a tincture, a concoction, an infusion a tablet, a capsule, a pill, a bar, a chewable gum, a lotion, a powder or granules.

10. A dietary composition comprising a herbal composition according to any of claims 1-9 and a dietetically acceptable excipient.

11. A pharmaceutical composition comprising a herbal composition according to any of claims 1-9 and a pharmaceutically acceptable excipient.

12. A food product which includes a composition according to any of claims 1-9.

13. The composition of any of claims 1-12, being packaged in a packaging material and identified in print, in or on said packaging material, for use in the treatment and/or prevention of diabetes and/or conditions associated therewith; and/or for use in the treatment and/or prevention of dyslipidemia.

14. Use of a composition according to any of claims 1-13, in the preparation of a medicament for treating and/or preventing diabetes and/or conditions associated therewith, and/or in the preparation of a medicament for treating and/or preventing dyslipidemia.

15. Use of a composition according to any one of claims 1-13 in the preparation of an orally-administrable composition, wherein the composition is a food supplement and/or wherein the composition is a pharmaceutical composition.

16. The use of claim 15, wherein said diabetes is type II diabetes.

17. The use of claim 16, wherein said type II diabetes associated conditions are selected from atherosclerosis, hypertension, diabetic retinopathy, diabetic nephropathy, diabetic polyneuropathies, thyroid disorders, leg ulcers, diabetic foot, liver diseases, kidney function, sight, impotence and constipation.

18. A process of preparing the composition of claim 1, wherein said extract of *Morus* species is prepared by:
a) cutting or grinding fresh washed leaves of at least one *Morus* species to obtain cut or ground fresh *Morus* leaves;
b) letting said cut or ground fresh *Morus* leaves stand until latex is exuded therefrom;
c) pressing said leaves to obtain a fresh juice and squeezed leaves;
d) collecting said juice;
e) brewing said juice to obtain a brewed juice;
f) chilling and filtering said brewed juice to obtain a liquid extract of at least one Morus species, and mixing thereto at least one *Urtica* species or an extract thereof, and at least one *Artemisia* species or an extract thereof.

19. A process of preparing the composition of claim 18, wherein said process further comprises the step of mixing thereto at least one species selected from a *Cinnamomum* species, a *Canella* species, a *Taraxacum* species and/or a Rosa species or extracts thereof.

20. A process of preparing the composition of claim 19, wherein said process further comprises the step of mixing thereto at least one species selected from a *Humulus* species, a *Gymnema* species, a *Trigonella* species, a *Punica* species, *a Salix* species, and/or an *Olea* species or extracts thereof.

## Patentansprüche

1. Eine pflanzliche Zusammensetzung, umfassend:
mindestens eine Spezies von *Urtica* oder einen Auszug einer solchen,
mindestens eine Spezies von *Artemisia* oder einen Auszug einer solchen,
einen Auszug von mindestens einer Spezies von *Morus,*
wobei der benannte Auszug aus einer Spezies von *Morus* aus den Blättern von *Morus* zubereitet wird und natürlichen Gummi von *Morus* umfasst.

2. Zusammensetzung nach Anspruch 1, bei welcher die benannte Spezies von *Morus* aus einer aus *Morus alba, Morus bombycis, Morus indica, Morus insignis, Morus nigra* und *Morus australis* bestehenden Gruppe und beliebigen Kombinationen derselben ausgewählt wird und/oder bei welcher die benannte Spezies von *Urtica* aus einer aus *Urtica dioica, Urtica urens* und *Urtica pilulifera* bestehenden Gruppe und beliebigen Kombinationen derselben ausgewählt wird und/oder bei welcher die benannte Spezies von *Artemisia* aus einer aus *Artemisia dracunculus, Artemisia herba alba, Artemisia pallens Wall, Artemisia roxburghiana* und *Artemisia judaica* bestehenden Gruppe und beliebigen Kombinationen derselben ausgewählt wird.

3. Zusammensetzung von Anspruch 1, bei welcher eine Menge des benannten Auszugs von *Morus* zwischen etwa 50 Gewichtsprozent und etwa 90 Gewichtsprozent des Gesamtgewichts der Zusammensetzung liegt, eine Menge der benannten Spezies von *Artemisia* zwischen etwa 1 Gewichtsprozent und etwa 20 Gewichtsprozent des Gesamtgewichts der Zusammensetzung liegt, und eine Menge der benannten Spezies von *Urtica* zwischen etwa 2 Gewichtsprozent und etwa 20 Gewichtsprozent des Gesamtgewichts der Zusammensetzung liegt.

4. Zusammensetzung nach Anspruch 1, welche ferner zumindest eine aus einer Spezies von *Cinnamomum,* aus einer Spezies von *Canella,* aus einer Spezies von *Taraxacum* und/oder aus einer Spezies von *Rosa* ausgewählte Spezies umfasst.

5. Zusammensetzung nach Anspruch 4, bei welcher die benannte Spezies von *Cinnamomum* aus einer aus *Cinnamomum cassia, Cinnamomum zeylanicum, Cinnamomum saigonicum, Cinnamomum aromaticum* und *Cinnamomum laurus* bestehenden Gruppe ausgewählt wird und/oder bei welcher die benannte Spezies von *Canella* die *Canella winterana* ist und/oder bei welcher die benannte Spezies von *Taraxacum* die *Taraxacum officinale* ist und/oder bei welcher die benannte Spezies von *Rosa* die *Rosa canina* ist.

6. Zusammensetzung von Anspruch 4, bei welcher eine Menge des benannten Auszugs von *Morus* zwischen etwa 50 Gewichtsprozent und etwa 90 Gewichtsprozent des Gesamtgewichts der Zusammensetzung liegt, eine Menge der benannten Spezies von *Artemisia* zwischen etwa 1 Gewichtsprozent und etwa 20 Gewichtsprozent des Gesamtgewichts der Zusammensetzung liegt, eine Menge der benannten Spezies von *Urtica* zwischen etwa 2 Gewichtsprozent und etwa 20 Gewichtsprozent des Gesamtgewichts der Zusammensetzung liegt, und eine Menge einer beliebigen aus einer Spezies von *Cinnamomum,* aus einer Spezies von *Canella,* aus einer Spezies von *Taraxacum* und/oder einer Spezies von Rosa ausgewählten Spezies zwischen etwa 5 Gewichtsprozent und etwa 30 Gewichtsprozent des Gesamtgewichts der Zusammensetzung liegt.

7. Zusammensetzung nach einem beliebigen der Ansprüche 1 - 6, ferner umfassend eine oder mehrere aus einer Spezies von *Humulus,* aus einer Spezies von *Gymnema,* aus einer Spezies von *Trigonella,* aus einer Spezies von *Punica,* aus einer Spezies von *Salix* und/oder aus einer Spezies von *Olea* ausgewählte Spezies.

8. Zusammensetzung nach einem beliebigen der Ansprüche 1 - 7, ferner umfassend mindestens einen Träger.

9. Zusammensetzung nach einem beliebigen der Ansprüche 1 - 8 in Form eines Tees, einer Tinktur, einer Zubereitung, einer Infusion, einer Tablette, einer Kapsel, einer Pille, eines Riegels, eines kaubaren Gummis, einer Lotion, eines Pulvers oder eines Granulats.

10. Eine Ernährungszusammensetzung, umfassend eine pflanzliche Zusammensetzung nach einem beliebigen der Ansprüche 1 - 9 und einen diätetisch verträglichen Hilfsstoff.

11. Eine pharmazeutische Zusammensetzung, umfassend eine pflanzliche Zusammensetzung nach einem beliebigen der Ansprüche 1 - 9 und einen pharmazeutisch verträglichen Hilfsstoff.

12. Ein Nahrungsmittel, das eine Zusammensetzung nach einem beliebigen der Ansprüche 1 - 9 einschließt.

13. Zusammensetzung nach einem beliebigen der Ansprüche 1 - 12, verpackt in Verpackungsmaterial und durch in oder auf dem benannten Verpackungsmaterial angebrachten Aufdruck zur Verwendung für die Behandlung von und/oder Vorbeugung gegen Diabetes und/oder mit Diabetes verbundene(n) Zustände(n) und/oder für die Behandlung von und/oder Vorbeugung gegen Dyslipidämie gekennzeichnet.

14. Verwendung einer Zusammensetzung nach einem beliebigen der Ansprüche 1 - 13 in der Herstellung eines Medikaments für die Behandlung von und/oder Vorbeugung gegen Diabetes und/oder mit Diabetes verbundene(n) Zustände(n) und/oder in der Herstellung eines Medikaments für die Behandlung von und/oder Vorbeugung gegen Dyslipidämie.

15. Verwendung einer Zusammensetzung nach einem beliebigen der Ansprüche 1 - 13 in der Herstellung einer oral verabreichbaren Zusammensetzung, bei welcher die Zusammensetzung ein Nahrungsergänzungsmittel ist und/oder bei welcher die Zusammensetzung eine pharmazeutische Zusammensetzung ist.

16. Die Verwendung nach Anspruch 15, bei welcher der benannte Diabetes Typ II Diabetes ist.

17. Die Verwendung nach Anspruch 16, bei welchem die benannten mit dem Typ II Diabetes verbundenen Zustände unter Arteriosklerose, Bluthochdruck, diabetischer Retinopathie, diabetischer Nephropathie, diabetischen Polyneuropathien, Erkrankungen der Schilddrüse, diabetischen Beingeschwüren, diabetischem Fuß, Lebererkrankungen, Nierenfunktion, Sicht, Impotenz und Obstipation ausgewählt werden.

18. Verfahren zur Herstellung der Zusammensetzung von Anspruch 1, bei welchem der benannte Auszug der Spezies von *Morus* wie folgt hergestellt wird:
a) Schneiden oder Zerreiben gewaschene frische Blätter von mindestens einer Spezies von *Morus,* um geschnittene oder zerriebene frische Blätter von *Morus* zu erhalten;
b) Ruhenlassen benannter geschnittener oder zerriebener Blätter von *Morus,* bis der natürliche Gummi aus ihnen abgesondert ist;
c) Pressen der benannten Blätter, um einen frischen Saft und ausgepresste Blätter zu erhalten;
d) Sammeln des benannten Saftes;
e) Brauen des benannten Saftes, um einen gebrauten Saft zu erhalten;
f) Kühlen und Filtern des benannten gebrauten Saftes, um einen flüssigen Auszug von mindestens einer Spezies von *Morus* zu erhalten, und Beimischen zumindest einer Spezies von *Urtica* oder eines Auszugs derselben und zumindest einer Spezies von *Artemisia* oder eines Auszugs derselben.

19. Verfahren zur Herstellung der Zusammensetzung von Anspruch 18, bei welchem das benannte Verfahren ferner den Schritt des Beimischens mindestens einer der aus einer Spezies von *Cinnamomum,* einer Spezies von *Canella,* einer Spezies von *Taraxacum* und/oder einer Spezies von Rosa ausgewählten Spezies oder von Auszügen derselben umfasst.

20. Verfahren zur Herstellung der Zusammensetzung von Anspruch 19, bei welchem das benannte Verfahren ferner den Schritt des Beimischens mindestens einer der aus einer Spezies von *Humulus,* einer Spezies von *Gymnema,* einer Spezies von *Trigonella,* einer Spezies von *Punica,* einer Spezies von *Salix* und/oder einer Spezies von *Olea* ausgewählten Spezies oder von Auszügen derselben umfasst.

## Revendications

1. Composition à base de plantes, comprenant :
au moins une espèce d*'Urtica* ou un extrait de celle-ci,
au moins une espèce *d'Artemisia* ou un extrait de celle-ci, et
un extrait d'au moins une espèce de *Morus,*
lequel extrait d'une espèce de *Morus* est préparé à partir de feuilles de *Morus* et comprend du latex de *Morus .*

2. Composition selon la revendication 1, dans laquelle ladite espèce de *Morus* est choisie dans le groupe constitué par *Morus alba, Morus bombycis, Morus indica, Morus insignis, Morus nigra* et *Morus australis* ainsi que des combinaisons quelconques de ces espèces, et/ou dans laquelle ladite espèce d*'Urtica* est choisie dans le groupe constitué par *Urtica dioica, Urtica urens* et *Urtica pilulifera* ainsi que des combinaisons quelconques de ces espèces, et/ou dans laquelle ladite espèce *d'Artemisia* est choisie dans le groupe constitué par *Artemisia dracunculus, Artemisia herba alba, Artemisia pallens Wall, Artemisia roxburghiana* et *Artemisia judaica* ainsi que des combinaisons quelconques de ces espèces.

3. Composition selon la revendication 1, dans laquelle la quantité dudit extrait de *Morus* va d'environ 50 % en poids à environ 90 % en poids, rapportée au poids total de la composition, la quantité de ladite espèce d*'Artemisia* va d'environ 1 % en poids à environ 20 % en poids, rapportée au poids total de la composition, et la quantité de ladite espèce d*'Urtica* va d'environ 2 % en poids à environ 20 % en poids, rapportée au poids total de la composition.

4. Composition selon la revendication 1, comprenant en outre au moins une espèce choisie parmi une espèce de *Cinnamomum,* une espèce de *Canella,* une espèce de *Taraxacum* et/ou une espèce de *Rosa.*

5. Composition selon la revendication 4, dans laquelle ladite espèce de *Cinnamomum* est choisie dans le groupe constitué par *Cinnamomum cassia, Cinnamomum zeylanicum, Cinnamomum saigonicum, Cinnamomum aromaticum* et *Cinnamomum laurus,* et/ou dans laquelle ladite espèce de *Canella* est *Canella winterana,* et/ou dans laquelle ladite espèce de *Taraxacum* est *Taraxacum officinale,* et/ou dans laquelle ladite espèce de *Rosa* est *Rosa canina.*

6. Composition selon la revendication 4, dans laquelle la quantité dudit extrait de *Morus* va d'environ 50 % en poids à environ 90 % en poids, rapportée au poids total de la composition, la quantité de ladite espèce d*'Artemisia* va d'environ 1 % en poids à environ 20 % en poids, rapportée au poids total de la composition, la quantité de ladite espèce d*'Urtica* va d'environ 2 % en poids à environ 20 % en poids, rapportée au poids total de la composition, et la quantité d'une éventuelle espèce choisie parmi une espèce de *Cinnamomum,* une espèce de *Canella,* une espèce de *Taraxacum* et/ou une espèce de *Rosa* va d'environ 5 % en poids à environ 30 % en poids, rapportée au poids total de la composition.

7. Composition selon l'une quelconque des revendications 1 à 6, comprenant en outre une ou plusieurs espèces choisies parmi une espèce d'*Humulus,* une espèce de *Gymnema,* une espèce de *Trigonella,* une espèce de *Punica,* une espèce de *Salix* et/ou une espèce d'*Olea*.

8. Composition selon l'une quelconque des revendications 1 à 7, comprenant en outre au moins un véhicule.

9. Composition selon l'une quelconque des revendications 1 à 8, sous forme de tisane, teinture, décoction, infusion, comprimé, gélule, pilule, barre, gomme à mâcher, lotion, poudre ou granulés.

10. Composition diététique comprenant une composition à base de plantes selon l'une quelconque des revendications 1 à 9 ainsi qu'un excipient acceptable du point de vue diététique.

11. Composition pharmaceutique comprenant une composition à base de plantes selon l'une quelconque des revendications 1 à 9 ainsi qu'un excipient acceptable du point de vue pharmaceutique.

12. Produit alimentaire comprenant une composition selon l'une quelconque des revendications 1 à 9.

13. Composition selon l'une quelconque des revendications 1 à 12, emballée dans un matériau d'emballage et identifiée par une impression dans ou sur ledit matériau d'emballage, destinée à être utilisée dans le traitement et/ou la prévention du diabète et/ou de conditions associées au diabète, et/ou destinée à être utilisée dans le traitement et/ou la prévention de la dyslipidémie.

14. Utilisation d'une composition selon l'une quelconque des revendications 1 à 13 dans la préparation d'un médicament destiné au traitement et/ou à la prévention du diabète et/ou de conditions associées au diabète, et/ou dans la préparation d'un médicament destiné au traitement et/ou à la prévention de la dyslipidémie.

15. Utilisation d'une composition selon l'une quelconque des revendications 1 à 13 dans la préparation d'une composition administrable par voie orale, dans laquelle la composition est un complément alimentaire et/ou dans laquelle la composition est une composition pharmaceutique.

16. Utilisation selon la revendication 15, dans laquelle ledit diabète est le diabète de type II.

17. Utilisation selon la revendication 16, dans laquelle lesdites conditions associées au diabète de type II sont choisies parmi l'athérosclérose, l'hypertension, la rétinopathie diabétique, la néphropathie diabétique, les polyneuropathies diabétiques, les troubles thyroïdiens, les ulcères des jambes, le pied diabétique, les maladies hépatiques, la fonction rénale, la vision, l'impuissance et la constipation.

18. Procédé de préparation de la composition selon la revendication 1, dans lequel ledit extrait de l'espèce *Morus* est préparé par les étapes consistant à :
a) hacher ou broyer des feuilles fraîches lavées d'au moins une espèce de *Morus* afin d'obtenir des feuilles de *Morus* fraîches hachées ou broyées ;
b) laisser reposer lesdites feuilles de *Morus* fraîches hachées ou broyées jusqu'à ce que le latex en ait exsudé ;
c) presser lesdites feuilles afin d'obtenir un jus frais et des feuilles pressées ;
d) recueillir ledit jus ;
e) brasser ledit jus afin d'obtenir un jus brassé ;
f) refroidir et filtrer ledit jus brassé afin d'obtenir un extrait liquide d'au moins une espèce de *Morus,*
et mélanger audit extrait au moins une espèce d*'Urtica* ou un extrait de celle-ci, et au moins une espèce d*'Artemisia* ou un extrait de celle-ci.

19. Procédé de préparation de la composition selon la revendication 18, lequel procédé comprend en outre l'étape consistant à mélanger à ladite préparation au moins une espèce choisie parmi une espèce de *Cinnamomum,* une espèce de *Canella,* une espèce de *Taraxacum* et/ou une espèce de *Rosa,* ou des extraits de ces espèces.

20. Procédé de préparation de la composition selon la revendication 19, lequel procédé comprend en outre l'étape consistant à mélanger à ladite préparation au moins une espèce choisie parmi une espèce d*'Humulus,* une espèce de *Gymnema,* une espèce de *Trigonella,* une espèce de *Punica,* une espèce de *Salix* et/ou une espèce d'*Olea*, ou des extraits de ces espèces.
